(19) Europäisches Patentamt
European Patent Office
Office européen des brevets

(11) **EP 2 346 949 B1**

(12) **EUROPÄISCHE PATENTSCHRIFT**

(45) Veröffentlichungstag und Bekanntmachung des Hinweises auf die Patenterteilung:
**17.07.2013 Patentblatt 2013/29**

(21) Anmeldenummer: **10742440.0**

(22) Anmeldetag: **09.08.2010**

(51) Int Cl.:
***C09C 1/00*** *(2006.01)*

(86) Internationale Anmeldenummer:
**PCT/EP2010/004866**

(87) Internationale Veröffentlichungsnummer:
**WO 2011/020571 (24.02.2011 Gazette 2011/08)**

(54) **HOCHGLÄNZENDE MEHRSCHICHTPERLGLANZPIGMENTE MIT NICHTSILBERNER INTERFERENZFARBE UND ENGER GRÖSSENVERTEILUNG UND VERFAHREN ZU DEREN HERSTELLUNG**

HIGH-GLOSS MULTILAYER EFFECT PIGMENTS HAVING A NON-SILVER INTERFERENCE COLOR AND A NARROW SIZE DISTRIBUTION, AND METHOD FOR THE PRODUCTION THEREOF

PIGMENTS NACRÉS MULTICOUCHE TRÈS BRILLANTS DOTÉS D'UNE TEINTE DE POLARISATION NON ARGENTÉE ET D'UNE ÉTROITE DISTRIBUTION GRANULOMÉTRIQUE ET LEUR PROCÉDÉ DE PRODUCTION

(84) Benannte Vertragsstaaten:
**AL AT BE BG CH CY CZ DE DK EE ES FI FR GB GR HR HU IE IS IT LI LT LU LV MC MK MT NL NO PL PT RO SE SI SK SM TR**

(30) Priorität: **19.08.2009 DE 102009037933**

(43) Veröffentlichungstag der Anmeldung:
**27.07.2011 Patentblatt 2011/30**

(73) Patentinhaber: **Eckart GmbH**
**91235 Hartenstein (DE)**

(72) Erfinder:
• **SCHUMACHER, Dirk**
**91257 Pegnitz (DE)**

• **GRÜNER, Michael**
**91275 Auerbach (DE)**
• **KAUPP, Günter**
**91284 Neuhaus (DE)**

(74) Vertreter: **Louis Pöhlau Lohrentz**
**Patentanwälte**
**Postfach 30 55**
**90014 Nürnberg (DE)**

(56) Entgegenhaltungen:
**WO-A1-2009/103322      WO-A2-2006/110359**
**US-A1- 2006 042 509**

**Beschreibung**

Die vorliegende Erfindung betrifft hochglänzende Mehrschichtperlglanzpigmente mit nichtsilberner Interferenzfarbe, ein Verfahren zu deren Herstellung sowie deren Verwendung in kosmetischen Formulierungen, Kunststoffen, Folien, Textilien, keramischen Materialien, Gläsern, Beschichtungszusammensetzungen wie Farben, Druckfarben, Tinten, Lacken oder Pulverlacken.

Goniochromatische Glanzpigmente werden in EP 0 753 545 B2 beschrieben. Mindestens ein Schichtpaket aus einer farblosen niedrigbrechenden und einer reflektierenden, selektiv oder nichtselektiv absorbierenden Beschichtung sowie optional einer äußeren Schutzschicht wird hier auf ein mehrfach beschichtetes, hochbrechendes, nichtmetallisches, plättchenförmiges Substrat aufgebracht. Die Schichtdicke der farblosen niedrigbrechenden Beschichtung verringert sich bei steigender Anzahl der auf dem Substrat aufgebrachten Schichtpakete. Die goniochromatischen Glanzpigmente zeigen einen winkelabhängigen Farbwechsel zwischen mehreren intensiven Interferenzfarben.

Gemäß WO 2004/067645 A2 wird ein transparentes Substrat mit einer ungeraden Anzahl, mindestens drei, abwechselnd hoch- und niedrigbrechenden Schichten beschichtet. Der Brechungsindexunterschied der aneinandergrenzenden Schichten liegt bei mindestens 0,2. Mindestens eine der Schichten unterscheidet sich in ihrer optischen Dicke von den anderen. Somit besitzen die resultierenden Mehrschichteffektpigmente keinen Schichtaufbau, bei dem die optische Dicke jeder Schicht ein ungeradzahliges Vielfaches eines Viertels der zu interferierenden Lichtwellenlänge beträgt (kein "quarter-wave-stack"-Aufbau).

In WO 2006/088759 A1 werden die Mehrschichteffektpigmente mit Titandioxid, einer niedrigbrechenden Schicht mit einer optischen Schichtdicke von mindestens 150 nm und anschließend wieder mit einer hochbrechenden Schicht umfassend Titandioxid mit einer optischen Schichtdicke von ca. 45 bis 240 nm beschichtet. Die erste Titandioxidschicht verleiht dem Substrat einen silbernen Glanz, während die resultierenden Mehrschichteffektpigmente jedoch keinen silbernen Glanz besitzen. Aufgrund der optischen Schichtdicke der niedrigbrechenden Schicht besitzen die Mehrschichteffektpigmente einen Farbflop. Analog zu WO 2004/067645 A2 weisen auch hier die aneinandergrenzenden Schichten einen Brechungsindexunterschied von mindestens 0,2 auf. Ebenfalls soll kein Schichtaufbau, bei dem die optische Dicke jeder Schicht ein ungeradzahliges Vielfaches eines Viertels der zu interferierenden Lichtwellenlänge beträgt (kein "quarter-wave-stack"-Aufbau) gegeben sein.

Mehrschichtige Interferenzpigmente mit kräftigen Interferenzfarben und/ oder einer starken Winkelabhängigkeit der Interferenzfarben, bestehend aus einem transparenten Trägermaterial, das mit alternierenden Schichten von Metalloxiden mit niedriger und hoher Brechzahl beschichtet ist, werden in EP 0 948 572 B1 beschrieben. Die Differenz der Brechzahlen beträgt mindestens 0,1. Die Anzahl und Dicke der Schichten ist abhängig vom gewünschten Effekt und dem verwendeten Substrat. Betrachtet man den Aufbau $TiO_2$-$SiO_2$-$TiO_2$ auf einem Glimmersubstrat, so lassen sich bei Verwendung optisch dünner $TiO_2$- und $SiO_2$-Schichten mit einer Schichtdicke < 100 nm beispielsweise Pigmente mit blauer Interferenzfarbe erhalten, die farbkräftiger als reine $TiO_2$-Glimmerpigmente sind. Durch die Auffällung dicker $SiO_2$-Schichten mit einer Schichtdicke > 100 nm werden Pigmente mit einer stark ausgeprägten Winkelabhängigkeit der Interferenzfarbe erhalten.

JP 07246366 beschreibt ein optisches Interferenzmaterial, das aus alternierenden hochbrechenden und niedrigbrechenden Schichten aufgebaut ist, wobei die optische Dicke jeder Schicht ein ungeradzahliges Vielfaches eines Viertels der zu interferierenden Lichtwellenlänge beträgt ("quater-wave-stack"-Aufbau).

Interferenzpigmente auf der Basis von mehrfach beschichteten plättchenförmigen Substraten, die mindestens eine Schichtenfolge aus einer hochbrechenden, einer farblosen niedrigbrechenden, einer nicht absorbierenden hochbrechenden und gegebenenfalls einer äußeren Schutzschicht aufweisen, können nach EP 1 025 168 B1 hergestellt werden. Zwischen dem Substrat und der ersten Schicht bzw. zwischen den einzelnen Schichten können sich weitere farbige oder farblose Metalloxidschichten befinden. Die Interferenzpigmente können mehrere, gleiche oder verschiedene Kombinationen an Schichtpaketen enthalten, bevorzugt ist aber die Belegung des Substrats mit nur einem Schichtpaket. Zur Intensivierung des Farbflops können die Interferenzpigmente bis zu vier Schichtpakete enthalten, wobei die Dicke aller Schichten auf dem Substrat 3 $\mu$m nicht überschreiten sollte.

Mehrschichtpigmente basierend auf Glasflakes, die mit mindestens drei alternierenden Schichten mit hohem und niedrigem Brechungsindex beschichtet sind, werden in WO 2003/006558 A2 beschrieben. Die Glasflakes besitzen hier eine Dicke von < 1$\mu$m. Die Mehrschichtpigmente weisen neben intensiven Farben einen starken Farbflop auf.

In WO 2004/055119 A1 werden Interferenzpigmente auf der Basis von beschichteten plättchenförmigen Substraten beschrieben. Die Substrate sind hierbei mit einer ersten Schicht aus $SiO_2$ belegt, auf die anschließend eine hochbrechende Schicht, bestehend aus beispielsweise $TiO_2$, $ZrO_2$, $SnO_2$, $Cr_2O_3$, $Fe_2O_3$ oder $Fe_3O_4$ bzw. ein Interferenzsystem aus alternierenden hoch- und niedrigbrechenden Schichten aufgebracht ist. Optional können die Pigmente noch eine äußere Schutzschicht aufweisen. Auf diese Weise werden silberweiße Interferenzpigmente bzw. Interferenzpigmente mit brillanten Interferenzfarben erhalten, die sich durch anwendungstechnische Eigenschaften, wie mechanische Stabilität und Photostabilität auszeichnen, allerdings keinen extrem hohen Glanz aufweisen. Die Interferenzpigmente zeigen keine oder nur eine geringe Winkelabhängigkeit der Farbe.

Thermisch und mechanisch stabile Effektpigmente basierend auf dünnen Glasplättchen mit einer Dicke $\leq 1,0$ $\mu$m sind aus WO 2002/090448 A2 bekannt. Die Effektpigmente können mit einer oder mehreren hoch- und/ oder niedrigbrechenden Schicht(en) belegt sein. Die Glasflakes besitzen eine Erweichungstemperatur von $\geq 800°C$.

Die optischen Eigenschaften von Effektpigmenten können nach WO 2006/110359 A2 durch eine geeignete Partikelgrößenverteilung beeinflusst werden. Die hier beschriebenen klassierten mit einer einzigen Metalloxidschicht beschichteten Glasplättchen weisen einen $D_{10}$ von wenigstens 9,5 $\mu$m, vorzugsweise von 9,5 $\mu$m, auf. Nachteilig ist, dass die Pigmente einen Größenbereich mit einem $D_{90}$-Wert von maximal 85 $\mu$m, vorzugsweise von etwa 45 $\mu$m, aufzuweisen haben.

Die US 2006/042509 A1 ist auf mit $SiO_2$ und Ceroxid beschichtete Perlglanzpigmente gerichtet.

Die WO 2009/103322 A1 ist ein Dokument gemäß Art. 54(3) EPÜ und beschreibt Effektpigmente basierend auf künstlich hergestellten Substraten mit enger Größenverteilung.

Der vorliegenden Erfindung liegt die Aufgabe zugrunde, hochglänzende Mehrschichtperlglanzpigmente mit nichtsilberner Interferenzfarbe und gleichzeitig hohem Chroma bereitzustellen, die den für Perlglanzpigmente typischen Tiefenglanz bei gleichzeitiger Transparenz aufweisen und die gegenüber den aus dem Stand der Technik bekannten Perlglanzpigmenten einen erhöhten Glanz besitzen. Weiterhin ist es Aufgabe der Erfindung, ein Verfahren zur Herstellung dieser Mehrschichtperlglanzpigmente zur Verfügung zu stellen.

Die der Erfindung zugrundeliegende Aufgabe wurde durch Bereitstellung von Mehrschichtperlglanzpigmenten gemäß Anspruch 1, umfassend mit optisch wirksamer Beschichtung versehene plättchenförmige transparente Substrate gelöst.

Bevorzugte Weiterbildungen sind in den abhängigen Ansprüchen 2 bis 11 angegeben.

Die der Erfindung zugrundeliegende Aufgabe wurde weiterhin gelöst durch Bereitstellung eines Verfahrens zum Herstellen der erfindungsgemäßen Mehrschichtperlglanzpigmente, welches folgende Schritte umfasst:

(i) Größenklassieren der zu beschichtenden plättchenförmigen transparenten Substrate, so dass die zu beschichtenden plättchenförmigen transparenten Substrate eine volumengemittelte Größenverteilungsfunktion mit den Kennzahlen $D_{10}$, $D_{50}$, $D_{90}$ und einem Span AD in einem Bereich von 0,7-1,4 aufweisen, wobei der Span $\Delta D$ gemäß der Formel $\Delta D = (D_{90} - D_{10})/ D_{50}$ definiert ist,

(ii) Aufbringen wenigstens der Schichten A bis C auf die plättchenförmigen transparenten Substrate sowie optional wenigstens einer Schicht D, oder

(iii) Aufbringen wenigstens der Schichten A bis C auf die plättchenförmigen transparenten Substrate sowie optional wenigstens einer Schicht D,

(iv) Größenklassieren der zu beschichtenden plättchenförmigen transparenten Substrate, so dass die zu beschichtenden plättchenförmigen transparenten Substrate eine volumengemittelte Größenverteilungsfunktion mit den Kennzahlen $D_{10}$, $D_{50}$, $D_{90}$ und einem Span $\Delta D$ in einem Bereich von 0,7 -1,4 aufweisen, wobei der Span $\Delta D$ gemäß der Formel $\Delta D = (D_{90} - D_{10})/ D_{50}$ definiert ist.

Vorzugsweise erfolgt das Beschichten der plättchenförmigen Substrate in Schritt (ii) nach dem Größenklassieren in Schritt (i).

Des Weiteren ist Gegenstand der Erfindung die Verwendung der erfindungsgemäßen Mehrschichtperlglanzpigmente in kosmetischen Formulierungen, Kunststoffen, Folien, Textilien, keramischen Materialien, Gläsern und Beschichtungszusammensetzungen, wie Farben, Druckfarben, Tinten, Lacken und Pulverlacken. Gegenstand der Erfindung sind mithin Zubereitungen, die die erfindungsgemäßen Mehrschichtperlglanzpigmente enthalten. Die Erfindung ist auch auf Gegenstände gerichtet, die mit den erfindungsgemäßen Mehrschichtperlglanzpigmenten versehen, beispielsweise lackiert oder bedruckt, sind. Somit sind lackierte Gegenstände, wie Karosserien, Fassadenelemente, etc. oder bedruckte Gegenstände, wie Papier, Pappe, Folien, Textilien, etc., ebenfalls Bestandteil der vorliegenden Erfindung.

Die Empfindung einer Farbe als matt, blass oder kräftig hängt maßgeblich von ihrer Farbsättigung, dem sogenannten Chroma oder der Buntheit ab. Dabei wird das Chroma durch die enthaltene Menge an Grau bestimmt. Je höher der Graugehalt, desto geringer ist die Farbsättigung.

Betrachtet man einen Punkt F im CIELab-Farbsystem, so ist dieser über die drei Koordinaten L* (Helligkeit), a* (rot-grün-Achse) und b* (gelb-blau-Achse) definiert. Die Farbkoordinaten a* und b* können auch über Polarkoordinaten C* (Chroma) und h* (Farbwinkel, Farbort) ausgedrückt werden, wobei die Definition wie folgt gegeben ist:

$$C^* = \sqrt{a^{*2} + b^{*2}}$$

$$h* = \frac{180}{\pi} \cdot \arctan\left(\frac{b*}{a*}\right)$$

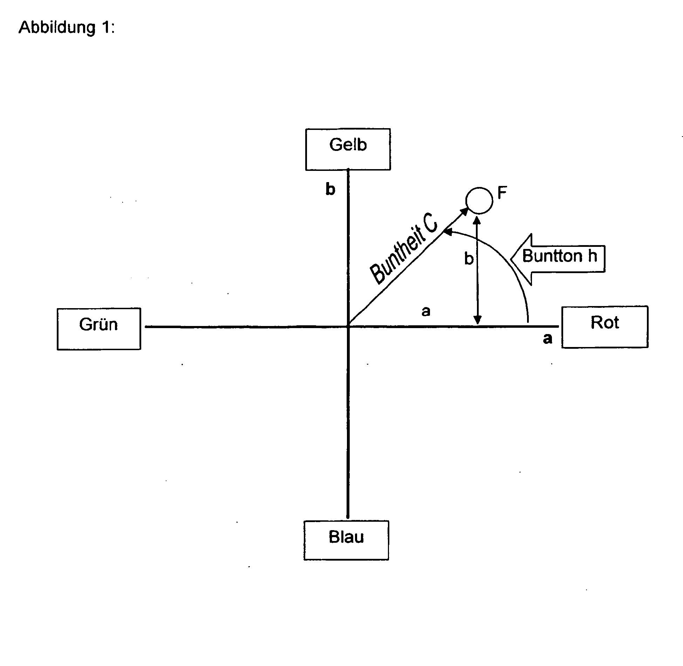

Das Chroma entspricht also der Länge des Vektors, der vom Ursprung des Koordinatensystems auf den zu definierenden Punkt F zeigt. Je geringer der C*-Wert ausfällt, desto näher liegt der Punkt F am unbunten, grauen Bereich des Farbkoordinatensystems. Das Chroma ist also der Abstand von der L*- oder Grau-Achse, die senkrecht auf der a*, b*-Ebene steht (Abbildung 1).

Effektpigmente, welche eine silberne Interferenzfarbe aufweisen, zeichnen sich durch geringe Chromawerte aus, d.h. es handelt sich um unbunte Interferenzfarben.

Unter Mehrschichtperlglanzpigmenten mit nichtsilberner Interferenzfarbe werden im Rahmen dieser Erfindung Mehrschichtperlglanzpigmente verstanden, deren Chromawerte $C*_{15} > 20$ sind.

Die Chromawerte werden dabei anhand von folgenden Applikationen ermittelt: Ein Nitrocelluloselack (Dr. Renger Erco Bronzemischlack 2615e; Fa. Morton), der 6 Gew.-% an Mehrschichtperlglanzpigmenten enthält, wobei sich die Gew.-% Angabe auf das Gesamtgewicht des Lackes bezieht, wird je nach $D_{50}$-Wert in einer Nassfilmdicke gemäß Tabelle 1 auf BYK-Gardner Schwarz-Weiß-Rakelkarten (Byko-Chart 2853) aufgetragen und nachfolgend bei Raumtemperatur getrocknet. An diesen Rakelkarten werden dann mit einem BYK-MAC (Fa. BYK Gardner) farbmetrische Auswertungen vorgenommen, wobei auf dem schwarzen Untergrund der Rakelkarte vermessen wird. Der Einfallswinkel beträgt 45° und herangezogen wird der Chromawert bei einem Beobachtungswinkel von 15°.

Tabelle 1: Nassfilmdicke in Abhängigkeit vom $D_{50}$-Wert der Mehrschichtperlglanzpigmente

| $D_{50}$-Wert | Spiralrakel |
| --- | --- |
| < 40 $\mu$m | 36 $\mu$m |
| 40 $\mu$m - 85 $\mu$m | 76 $\mu$m |
| > 85 $\mu$m | 100 $\mu$m |

Die Erfinder haben überraschend festgestellt, dass die erfindungsgemäßen Mehrschichtperlglanzpigmente mit einem Span $\Delta D = (D_{90} - D_{10})/D_{50}$ im Bereich von 0,7 bis 1,4 einen außerordentlich starken Glanz und ein signifikant erhöhtes Chroma aufweisen.

Zur Charakterisierung der Größenverteilung der Mehrschichtperlglanzpigmente wird erfindungsgemäß der Span $\Delta D$, definiert als $\Delta D = (D_{90} - D_{10})/D_{50}$, verwendet. Je kleiner der Span ist, desto enger ist die Größenverteilung.

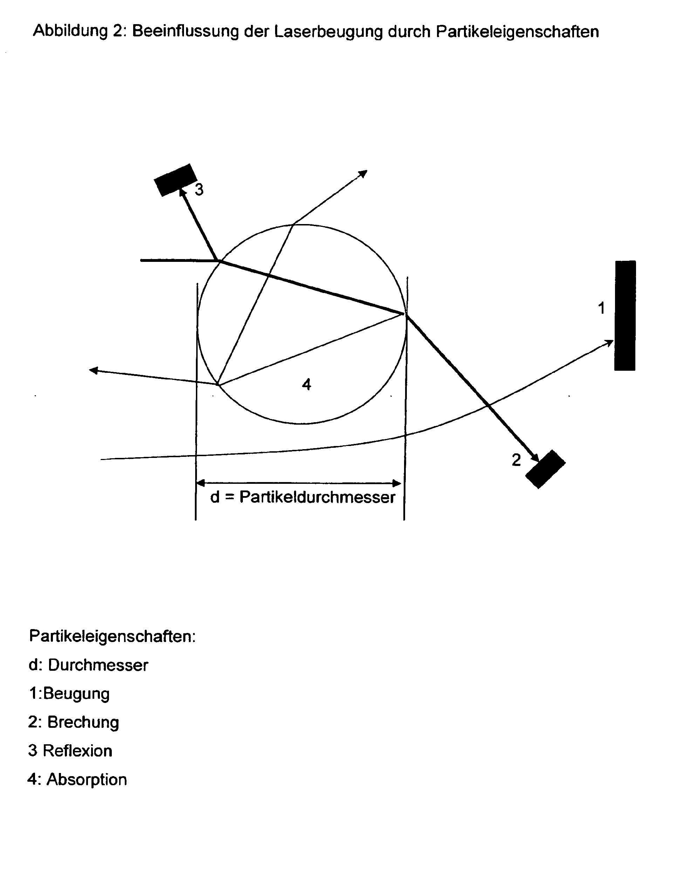

Der $D_{10}$-, $D_{50}$- bzw. $D_{90}$-Wert der Summenhäufigkeitsverteilung der volumengemittelten Größenverteilungsfunktion, wie sie durch Laserbeugungsmethoden erhalten wird, gibt an, dass 10%, 50% bzw. 90% der Mehrschichtperlglanzpigmente einen Durchmesser aufweisen, der gleich oder kleiner als der jeweils angegebene Wert, ist. Hierbei wird die Größenverteilungskurve mit einem Gerät der Fa. Malvern (Gerät: MALVERN Mastersizer 2000) gemäß Herstellerangaben bestimmt. Die Auswertung der Streulichtsignale erfolgte dabei nach der Mie-Theorie, welche auch Brechungsund Absorptionsverhalten der Partikel beinhaltet (Abbildung 2).

Die erfindungsgemäßen Mehrschichtperlglanzpigmente besitzen einen Span $\Delta D$ in einem Bereich von 0,7 bis 1,4, vorzugsweise in einem Bereich von 0,7 bis 1,3, weiterhin bevorzugt in einem Bereich von 0,8 bis 1,2, besonders bevorzugt in einem Bereich von 0,8 bis 1,1. Bei weiterhin bevorzugten Ausführungsformen liegt der Span $\Delta D$ in einem Bereich von 0,85 bis 1,05.

Weisen die Mehrschichtperlglanzpigmente einen Span $\Delta D$ oberhalb von 1,4 auf, so werden keine hochglänzenden Mehrschichtperlglanzpigmente erhalten. Mehrschichtperlglanzpigmente unterhalb eines Spans der Größenverteilung von 0,7 können im Rahmen der üblichen Methoden nur sehr aufwändig und damit nicht mehr wirtschaftlich hergestellt werden.

Der Span $\Delta D$ des zu beschichtenden, plättchenförmigen transparenten Substrats entspricht im Wesentlichen dem des erfindungsgemäßen Mehrschichtperlglanzpigments und liegt bei $\leq 1,4$, vorzugsweise $\leq 1,3$, weiter bevorzugt $\leq 1,2$, besonders bevorzugt $\leq 1,1$ und ganz besonders bevorzugt bei $\leq 1,05$.

Die erfindungsgemäßen Mehrschichtperlglanzpigmente können eine beliebige mittlere Teilchengröße ($D_{50}$) aufweisen. Die $D_{50}$-Werte der erfindungsgemäßen Mehrschichtperlglanzpigmente liegen vorzugsweise in einem Bereich von 3 bis 350 $\mu$m. Bevorzugt weisen die erfindungsgemäßen Mehrschichtperlglanzpigmente einen $D_{50}$-Wert aus einem Bereich von 3 bis 15 $\mu$m oder aus einem Bereich von 10 bis 35 $\mu$m oder aus einem Bereich von 25 bis 45 $\mu$m oder aus einem Bereich von 30 bis 65 $\mu$m oder aus einem Bereich von 40 bis 140 $\mu$m oder aus einem Bereich von 135 bis 250 $\mu$m auf.

Die $D_{10}$-Werte der erfindungsgemäßen Mehrschichtperlglanzpigmente umfassen vorzugsweise einen Bereich von 1 bis 120 μm. Bevorzugt weisen die erfindungsgemäßen Mehrschichtperlglanzpigmente die in Tabelle 2 angegebenen Kombinationen von $D_{10}$-, $D_{50}$- und $D_{90}$-Werten auf. Hierbei werden die $D_{10}$, $D_{50}$ und $D_{90}$-Werte der Tabelle 2 nur in der Art kombiniert, dass sich ein Span $\Delta D$ aus einem Bereich von 0,7 bis 1,4, vorzugsweise aus einem Bereich von 0,7 bis 1,3, weiterhin bevorzugt aus einem Bereich von 0,8 bis 1,2, besonders bevorzugt aus einem Bereich von 0,8 bis 1,1 und ganz besonders bevorzugt aus einem Bereich von 0,85 bis 1,05 ergibt. Kombinationen von $D_{10}$-, $D_{50}$- und $D_{90}$-Werten, die zu einem Span $\Delta D$ führen, der nicht im Bereich von 0,7 bis 1,4 liegt, sind keine erfindungsgemäßen Ausführungsformen.

Tabelle 2: Bevorzugte Kombinationen von Bereichen der $D_{10}$-, $D_{50}$- und $D_{90}$-Werte

| $D_{10}$ (μm) | $D_{50}$ (μm) | $D_{90}$ (μm) |
|---|---|---|
| 1 - 5 | 3 - 15 | 8 - 25 |
| 5 - 25 | 10 - 35 | 20 - 45 |
| 10 - 30 | 25 - 45 | 40 - 70 |
| 20 - 45 | 30 - 65 | 70 - 110 |
| 25 - 65 | 40 - 140 | 120 - 180 |
| 75 - 110 | 135 - 250 | 400 - 490 |

Dabei hat sich überraschenderweise gezeigt, dass die Größe der Mehrschichtperlglanzpigmente, charakterisiert durch den $D_{50}$- Wert, nicht entscheidend ist, sondern dass der Span $\Delta D = (D_{90} - D_{10})/ D_{50}$ in einem engen Bereich von 0,7 bis 1,4 liegt. Dabei können beispielsweise die $D_{50}$-Werte der Mehrschichtperlglanzpigmente bei 15, 20, 25, 30 μm oder auch bei 50, 80, 100, 150, 200, 250, 300 oder 350 μm liegen.

Geeignete zu beschichtende plättchenförmige transparente Substrate sind nichtmetallische, natürliche oder synthetische plättchenförmige Substrate. Die Substrate sind vorzugsweise im Wesentlichen transparent, bevorzugt transparent, d.h. für sichtbares Licht sind sie zumindest teilweise durchlässig.

Gemäß einer bevorzugten Ausführungsform der Erfindung können die plättchenförmigen transparenten Substrate aus der Gruppe, bestehend aus natürlichem Glimmer, synthetischem Glimmer, Glasflakes, $SiO_2$-Plättchen, $Al_2O_3$-Plättchen, Polymerplättchen, plättchenförmigem Bismuthoxychlorid, plättchenförmigen Substraten umfassend eine anorganisch-organische Mischschicht und deren Gemische, ausgewählt werden. Bevorzugt werden die plättchenförmigen transparenten Substrate aus der Gruppe, bestehend aus natürlichem Glimmer, synthetischem Glimmer, Glasflakes, $SiO_2$-Plättchen, $Al_2O_3$-Plättchen und deren Gemische, ausgewählt. Besonders bevorzugt werden die plättchenförmigen transparenten Substrate aus der Gruppe, bestehend aus natürlichem Glimmer, synthetischem Glimmer, Glasflakes und deren Gemische, ausgewählt. Ganz besonders bevorzugt sind Glasflakes und synthetischer Glimmer und deren Gemische.

Natürlicher Glimmer besitzt im Unterschied zu synthetischen plättchenförmigen transparenten Substraten den Nachteil, dass Verunreinigungen durch eingelagerte Fremdionen den Farbton verändern können, und dass die Oberfläche nicht ideal glatt ist, sondern Unregelmäßigkeiten, wie z.B. Stufen aufweisen kann.

Jedoch auch bei Verwendung von natürlichem Substrat hat sich überraschenderweise gezeigt, dass der Glanz einer Mehrzahl von Mehrschichtperlganzpigmenten gesteigert werden kann, wenn der Span $\Delta D$ in einem Bereich von 0,7 bis 1,4 liegt, verglichen mit einer Mehrzahl an herkömmlichen Mehrschichtperlglanzpigmenten mit breitem Span.

Synthetische Substrate wie beispielsweise Glasflakes oder synthetischer Glimmer dagegen weisen glatte Oberflächen, eine einheitliche Dicke innerhalb eines einzelnen Substratteilchens sowie scharfe Kanten auf. Somit bietet die Oberfläche nur wenig Streuzentren für einfallendes bzw. reflektiertes Licht und ermöglicht so nach Beschichtung höher glänzende Mehrschichtperlglanzpigmente als mit natürlichem Glimmer als Substrat. Als Glasflakes werden bevorzugt jene verwendet, die nach den in EP 0 289 240 A1, WO 2004/056716 A1 und der WO 2005/063637 A1 beschriebenen Verfahren hergestellt werden. Die als Substrat verwendbaren Glasflakes können beispielsweise eine Zusammensetzung entsprechend der Lehre der EP 1 980 594 B1 aufweisen.

Die mittlere geometrische Dicke der zu beschichtenden plättchenförmigen transparenten Substrate liegt in einem Bereich von 50 nm bis 5000 nm, bevorzugt in einem Bereich von 60 nm bis 3000 nm und besonders bevorzugt in einem Bereich von 70 nm bis 2000 nm. Weitere Vorteile bieten dünnere Glasflakes. Dünnere Substrate führen zu einer geringeren Gesamtschichtdicke der erfindungsgemäßen Mehrschichtperlglanzpigmente. Erfindungsgemäß verwendete Glasflakes weisen eine-mittlere geometrische Dicke in einem Bereich von von 100 nm bis 700 nm, weiter bevorzugt in einem Bereich von 150 nm bis 600 nm, besonders bevorzugt in einem Bereich von 170 nm bis 500 nm und ganz besonders bevorzugt in einem Bereich von 200 nm bis 400 nm auf. In einer weiteren Ausführungsform liegt die mittlere geometrische Dicke für natürlichen oder synthetischen Glimmer als zu beschichtendes Substrat bevorzugt in einem Bereich von 100 nm bis 700 nm, weiter bevorzugt in einem Bereich von 150 nm bis 600 nm, besonders bevorzugt in einem Bereich von 170 nm bis 500 nm und ganz besonders bevorzugt in einem Bereich von 200 nm bis 400 nm.

Beschichtet man plättchenförmige transparente Substrate unterhalb einer mittleren geometrischen Dicke von 50 nm mit hochbrechenden Metalloxiden, so werden extrem bruchempfindliche Mehrschichtperlglanzpigmente erhalten, die schon beim Einarbeiten in das Anwendungsmedium zerbrechen können, was wiederum eine signifikante Herabsetzung des Glanzes bedingt.

Oberhalb einer mittleren geometrischen Substratdicke von 5000 nm können die Mehrschichtperlglanzpigmente insgesamt zu dick werden. Damit geht eine schlechtere spezifische Deckfähigkeit, d.h. abgedeckte Fläche pro Gewichtseinheit an erfindungsgemäßem Mehrschichtperlglanzpigment, einher sowie eine geringere planparallele Orientierung im Anwendungsmedium. Aus einer schlechteren Orientierung wiederum resultiert ein verminderter Glanz.

Die mittlere geometrische Dicke des plättchenförmigen transparenten Substrates wird anhand eines gehärteten Lackfilmes, in dem die Mehrschichtperlglanzpigmente im Wesentlichen planparallel zum Untergrund ausgerichtet sind, bestimmt. Hierzu wird ein Querschliff des gehärteten Lackfilmes unter einem Rasterelektronenmikroskop (REM) untersucht, wobei die geometrische Dicke des plättchenförmigen transparenten Substrates von 100 Mehrschichtperlglanzpigmenten bestimmt und statistisch gemittelt wird.

Die erfindungsgemäßen Mehrschichtperlglanzpigmente werden durch Beschichtung der plättchenförmigen transparenten Substrate mit wenigstens einer optisch wirksamen Beschichtung erhalten, welche

a) eine nicht absorbierende hochbrechende Schicht A mit einem Brechungsindex n ≥ 1,8
b) eine niedrigbrechende Schicht B mit einem Brechungsindex n < 1,8
c) eine nicht absorbierende hochbrechende Schicht C mit einem Brechungsindex n ≥ 1,8
sowie
d) optional eine äußere Schutzschicht D umfasst. Die Schichten A und B bzw. B und C können unter der äußeren Schutzschicht D mehrfach aufgebracht werden.

Bevorzugt werden immer alternierend hoch- und niedrigbrechende Schichten auf dem Substrat aufgebracht. Besonders bevorzugt wird das plättchenförmige Substrat nur einmal mit den Schichten A bis C, optional D beschichtet.

Erfindungsgemäß ist die Schicht A in der Schichtenanordnung innenliegend, d.h. dem plättchenförmigen transparenten Substrat zugewandt, die Schicht B zwischen der Schicht A und der Schicht C liegend, und die Schicht C, bezogen auf das plättchenförmige transparente Substrat, in der Schichtenanordnung außenliegend.

Zwischen dem plättchenförmigen transparenten Substrat und der Schicht A können eine oder mehrere weitere, vorzugsweise im Wesentlichen transparente Schichten, angeordnet sein. Gemäß einer bevorzugten Weiterbildung ist die Schicht A direkt auf dem plättchenförmigem transparenten Substrat aufgebracht.

Zwischen der Schicht A und der Schicht B sowie zwischen der Schicht B und der Schicht C können unabhängig voneinander eine oder mehrere weitere, vorzugsweise im Wesentlichen transparente Schichten, angeordnet sein. Gemäß einer bevorzugten Weiterbildung ist die Schicht B direkt auf der Schicht A aufgebracht. Gemäß einer weiteren bevorzugten Weiterbildung ist die Schicht C direkt auf der Schicht B aufgebracht.

Äußerst bevorzugt ist die Schicht A unmittelbar auf dem plättchenförmigen transparenten Substrat, die Schicht B unmittelbar auf der Schicht A und die Schicht C unmittelbar auf der Schicht B sowie optional Schicht D unmittelbar auf der Schicht C aufgebracht.

Als optische wirksame Schichten oder Beschichtungen werden vorzugsweise Schichten aufgebracht, die Metalloxide, Metalloxidhydrate, Metallhydroxide, Metallfluoride, Metalloxyhalide, Metallchalcogenide, Metallsulfide oder Mischungen davon umfassen. Gemäß einer bevorzugten Variante bestehen die optisch wirksamen Schichten oder Beschichtungen aus den vorstehend genannten Materialien.

Die Begriffe Schichten oder Beschichtungen werden im Sinne dieser Erfindung austauschbar verwendet, sofern nicht anders angegeben.

Der Brechungsindex der hochbrechenden Schichten A und C liegt jeweils bei n ≥ 1,8, bevorzugt bei n ≥ 1,9 und besonders bevorzugt bei n ≥ 2,0. Der Brechungsindex der niedrigbrechenden Schicht B liegt bei n < 1,8, bevorzugt bei n < 1,7 und besonders bevorzugt bei n < 1,6.

Für die hochbrechende Schicht A bzw. C eignen sich als nicht absorbierende Materialien beispielsweise

■ Metalloxide wie Titandioxid, Zirkoniumdioxid, Zinkoxid, Zinndioxid, Antimonoxid und deren Gemische,
■ Metallhydroxide,
■ Metalloxidhydrate,
■ Metallsulfide wie Zinksulfid,
■ Metalloxyhalide wie Bismutoxychlorid.

Bei einer weiteren Ausführungsform kann die hochbrechende Metalloxidbeschichtung geringe Mengen (im Allgemeinen ≤ 5 Gew.%) weiterer farbiger oder farbloser Metalloxide enthalten. Beispielsweise kann eine Titandioxidschicht mit geringen Mengen an Eisenoxid dotiert sein.

In einer bevorzugten Ausführungsform werden als hochbrechende Schicht A bzw. C Metalloxide, Metallhydroxide und/ oder Metalloxidhydrate verwendet. Besonders bevorzugt werden Metalloxide eingesetzt. Ganz besonders bevorzugt umfassen die Schichten A und C Titandioxid. In einer Ausführungsform besteht die Schicht A und C aus Titandioxid.

Das Titandioxid kann hierbei in der Rutil- oder Anataskristallmodifikation vorliegen. Vorzugsweise liegt die Titandioxidschicht in der Rutilform vor. Die Rutilform kann erhalten werden, indem beispielsweise vor Aufbringung der Titandioxidschicht eine Schicht aus Zinndioxid auf das zu beschichtende Substrat aufgebracht wird. Auf dieser Schicht aus Zinndioxid kristallisiert Titandioxid in der Rutilmodifikation. Das Zinndioxid kann dabei als separate Schicht vorliegen, wobei die Schichtdicke wenige Nanometer, beispielsweise weniger als 10 nm, weiter bevorzugt weniger als 5 nm, noch weiter bevorzugt weniger als 3 nm, betragen kann.

Als niedrigbrechende Schicht B werden nicht absorbierende Materialien eingesetzt. Hierzu zählen beispielsweise

- Metalloxide wie Siliziumdioxid, Aluminiumoxid, Boroxid,
- Metalloxidhydrate wie Siliziumoxidhydrat, Aluminiumoxidhydrat,
- Metallfluoride wie Magnesiumfluorid,
- $MgSiO_3$.

Gegebenenfalls kann die niedrigbrechende Metalloxidschicht Alkali- und /oder Erdalkalioxide als Bestandteile enthalten.

Vorzugsweise umfasst die niedrigbrechende Schicht B Siliziumdioxid. In einer Ausführungsform besteht die niedrigbrechende Schicht B aus Siliziumdioxid.

Die interferenzfähige Beschichtung kann entweder das Substrat vollständig umhüllen oder nur partiell auf dem Substrat vorliegen. Die erfindungsgemäßen Mehrschichtperlglanzpigmente zeichnen sich durch den gleichmäßigen homogenen Aufbau der Beschichtung aus, welche das plättchenförmige Substrat vollständig umhüllt und nicht nur dessen Ober- und Unterseite bedeckt.

Die einzelnen Schichten der erfindungsgemäßen Mehrschichtperlglanzpigmente können jeweils als λ/4-Schichten ausgebildet sein. Es hat sich jedoch überraschend herausgestellt, dass es nicht erforderlich ist, dass die Schichten als λ/4-Schichten ausgebildet sein müssen, um glanzstarke und vorzugsweise auch hochchromatische Mehrschichtperlglanzpigmente zu erhalten. Der entscheidende Parameter ist der Span der Größenverteilung, um glanzstarke und vorzugsweise auch Mehrschichtperlglanzpigmente mit hohem Chroma zu erhalten.

Die optische Dicke der nichtmetallischen Schichten mit hoher und niedriger Brechzahl bestimmt die optischen Eigenschaften der Mehrschichtperlglanzpigmente. Die Anzahl und Dicke der Schichten ist abhängig vom gewünschten Effekt und vom verwendeten Substrat. Wenn n die Brechzahl einer Schicht und d ihre Dicke ist, ergibt sich die Interferenzfarbe, in der eine dünne Schicht erscheint, aus dem Produkt von n und d, d.h. der optischen Dicke. Die bei Normallichteinfall im reflektierenden Licht entstehenden Farben eines solchen Filmes ergeben sich aus einer Verstärkung des Lichtes der Wellenlänge

$$\lambda = \frac{4}{2N-1} \cdot nd$$

und durch Schwächung von Licht der Wellenlänge

$$\lambda = \frac{2}{N} \cdot nd \, ,$$

wobei N eine positive ganze Zahl ist. Die bei zunehmender Filmdicke erfolgende Variation der Farbe ergibt sich aus der Verstärkung bzw. Schwächung bestimmter Wellenlängen des Lichtes durch Interferenz.

Bei mehrschichtigen Pigmenten wird die Interferenzfarbe durch die Verstärkung bestimmter Wellenlängen bestimmt und wenn mehrere Schichten in einem vielschichtigen Pigment die gleiche optische Dicke besitzen, wird die Farbe des reflektierenden Lichtes mit zunehmender Zahl der Schichten intensiver. Darüber hinaus kann durch geeignete Wahl der Schichtdicke der niedrigbrechenden Schicht B eine besonders starke Variation der Farbe in Abhängigkeit vom Betrachtungswinkel erreicht werden. Es kann sich somit ein ausgeprägter Farbflop ausbilden.

Die erfindungsgemäßen Mehrschichtpigmente mit nichtsilberner Interferenzfarbe können optische Schichtdicken der hochbrechenden Schichten A und C aufweisen, die jeweils im Bereich von 30 nm bis 900 nm liegen. bevorzugt im Bereich von 40 nm bis 880 nm und besonders bevorzugt im Bereich von 50 nm bis 850 nm liegen. Die optische Schichtdicke der niedrigbrechenden Schicht B kann in einem Bereich von 30 nm bis 500 liegen.

Gemäß einer bevorzugten Ausführungsform der vorliegenden Erfindung weist bei den vorstehend genannten Schichtenfolgen die Schicht B eine optische Schichtdicke von ≤ 150 nm, vorzugsweise von < 140 nm und noch weiter bevorzugt von < 130 nm auf. Eine optische Schichtdicke der Schicht B im Bereich von 30 nm bis ≤ 150 nm, bevorzugt im Bereich von 40 nm bis 140 nm und besonders bevorzugt im Bereich von 50 nm bis 130 nm hat sich als sehr geeignet erwiesen.

Wenn die optische Schichtdicke der Schicht B ≤ 150 nm ist, weisen die erfindungsgemäßen Mehrschichtperlglanzpigmente im Wesentlichen keine winkelabhängige Interferenzfarbe auf. Bei dieser Ausführungsform weisen die erfindungsgemäßen Mehrschichtperlglanzpigmente nur eine Interferenzfarbe auf, wobei sich deren Intensität von hell nach dunkel in Abhängigkeit vom Betrachtungswinkel ändert.

Gemäß einer weiteren bevorzugten Weiterbildung der vorliegenden Erfindung weist bei den vorstehend genannten Schichtenfolgen die Schicht B eine optische Schichtdicke von > 150 nm, vorzugsweise von > 180 nm, noch weiter bevorzugt von > 220 nm, auf. Eine optische Schichtdicke der Schicht B im Bereich von > 150 nm bis 500 nm, bevorzugt im Bereich von 180 nm bis 480 nm, und besonders bevorzugt im Bereich von 220 nm bis 450 nm hat sich als sehr geeignet erwiesen.

Wenn die optische Schichtdicke der Schicht B > 150 nm ist, weisen die erfindungsgemäßen Mehrschichtperlglanzpigmente eine winkelabhängige Interferenzfarbe auf. Bei dieser Ausführungsform weisen die erfindungsgemäßen Mehrschichtperlglanzpigmente wenigstens zwei Interferenzfarben in Abhängigkeit vom Betrachtungswinkel auf. Diese Ausführungsform der erfindungsgemäßen Mehrschichtperlglanzpigmente kann auch als goniochromatisches Mehrschichtperlglanzpigment bezeichnet werden. Bei dieser Variante der Erfindung werden mithin hochglänzende Mehrschichtperlglanzpigmente mit intensivem Farbflop erhalten. Beispielsweise können diese Mehrschichtperlglanzpigmente einen Interferenzfarbwechsel von rot nach grün oder von blau nach gelb aufweisen.

Der Übergang zwischen Mehrschichtperlglanzpigmenten mit keinem, schwachem und intensivem Farbflop in Abhängigkeit von der optischen Schichtdicke der niedrigbrechenden Schicht B verläuft fließend. Mit zunehmender optischer Schichtdicke der niedrigbrechenden Schicht B werden ab 150 nm zunächst Mehrschichtperlglanzpigmente erhalten, die nur einen schwachen Farbflop aufweisen, welcher letztendlich mit immer weiter steigender optischen Schichtdicke von Schicht B in einen intensiven Farbflop übergeht. Typischerweise erstreckt sich ein intensiver Farbflop über mehrere Quadranten im CIELab-Farbkoordinatensystem.

Bei den in dieser Anmeldung angegebenen Schichtdicken handelt es sich - sofern nicht anders angegeben - um die optischen Schichtdicken. Unter einer optischen Schichtdicke wird erfindungsgemäß das Produkt aus geometrischer Schichtdicke und dem Brechungsindex des die Schicht aufbauenden Materials verstanden. Als Wert für den Brechungsindex des jeweiligen Materials wird der aus der Literatur jeweils bekannte Wert verwendet. Die geometrische Schichtdicke wird dabei erfindungsgemäß anhand von Querschliffaufnahmen von Lacken, in denen planparallel zum Untergrund orientierte Mehrschichtperlglanzpigmente vorliegen, mittels REM bestimmt.

Die Mehrschichtperlglanzpigmente können weiterhin mit wenigstens einer äußeren Schutzschicht D versehen sein, die die Licht-, Wetter-, und/ oder chemische Stabilität des Mehrschichtperlglanzpigmentes weiter erhöht. Bei der äußeren Schutzschicht D kann es sich auch um eine Nachbeschichtung handeln, die die Handhabung des Pigments bei Einarbeitung in unterschiedliche Medien erleichtert.

Die äußere Schutzschicht D der erfindungsgemäßen Mehrschichtperlglanzpigmente kann eine oder zwei Metalloxidschichten der Elemente Si, Al oder Ce umfassen bzw. bevorzugt daraus bestehen. Bei einer Variante wird als äußerste Metalloxidschicht eine Siliziumoxidschicht, vorzugsweise $SiO_2$-Schicht, aufgebracht. Besonders bevorzugt ist hierbei eine Reihenfolge, bei der zunächst eine Ceroxidschicht aufgebracht ist, der dann eine $SiO_2$-Schicht folgt, wie in der WO 2006/021386 A1 beschrieben, deren Inhalt hiermit unter Bezugnahme aufgenommen ist.

Die äußere Schutzschicht D kann des Weiteren an der Oberfläche organischchemisch modifiziert sein. Beispielsweise können ein oder mehrere Silane auf dieser äußeren Schutzschicht aufgebracht sein. Bei den Silanen kann es sich um Alkylsilane mit verzweigtkettigen oder unverzweigten Alkylresten mit 1 bis 24 C-Atomen, vorzugsweise 6 bis 18 C-Atomen handeln.

Bei den Silanen kann es sich aber auch um organofunktionelle Silane handeln, die eine chemische Anbindung an einen Kunststoff, ein Bindemittel eines Lackes oder einer Farbe, etc. ermöglichen.

Die vorzugsweise als Oberflächenmodifizierungsmittel verwendeten organofunktionellen Silane, die geeignete funktionelle Gruppen aufweisen, sind kommerziell verfügbar und werden beispielsweise von der Fa. Evonik hergestellt und unter dem Handelsnamen "Dynasylan" vertrieben. Weitere Produkte können von der Fa. Momentive (Silquest-Silane) oder von der Fa. Wacker, beispielsweise Standard- und α-Silane aus der GENIOSIL-Produktgruppe, bezogen werden. Beispiele hierfür sind 3-Methacryloxypropyltrimethoxysilan (Dynasylan MEMO, Silquest A-174NT), Vinyltri(m)ethoxysilan (Dynasylan VTMO bzw. VTEO, Silquest A-151 bzw. A-171), Methyltri(m)ethoxysilan (Dynasylan MTMS bzw.

MTES), 3-Mercaptopropyltrimethoxysilan (Dynasylan MTMO; Silquest A-189), 3-Glycidoxypropyltrimethoxysilan (Dynasylan GLYMO, Silquest A-187), Tris[3-(trimethoxysilyl)propyl]isocyanurat (Silquest Y-11597), Bis[3-(triethoxysilyl)propyl]tetrasulfid (Silquest A-1289), Bis[3-(triethoxysilyl)propyldisulfid (Silquest A-1589), beta-(3,4-Epoxycyclohexyl) ethyltrimethoxysilan (Silquest A-186), Bis(triethoxysilyl)ethan (Silquest Y-9805), gamma-Isocyanatopropyltrimethoxysilan (Silquest A-Link 35, GENIOSIL GF40), Methacryloxymethyltri(m)ethoxysilan (GENIOSIL XL 33, XL 36), (Methacryloxymethyl)(m)ethyldimethoxysilan (GENIOSIL XL 32, XL 34), (Isocyanatomethyl)methyldimethoxysilan,

(Isocyanatomethyl)trimethoxysilan, 3-(Triethoxysilyl)propylbernsteinsäureanhydrid (GENIOSIL GF 20), (Methacryloxymethyl)methyldiethoxysilan, 2-Acryloxyethylmethyldimethoxysilan, 2-Methacryloxyethyltrimethoxysilan, 3-Acryloxypropylmethyldimethoxysilan, 2-Acryloxyethyltrimethoxysilan, 2-Methacryloxyethyltriethoxysitan, 3-Acryloxypropyltrimethoxysilan, 3-Acryloxypropyltripropoxysilan, 3-Methacryloxypropyltriethoxysilan, 3-Methacryloxypropyltriacetoxysilan, 3- Methacryloxypropylmethyldimethoxysilan, Vinyltrichlorsilan, Vinyltrimethoxysilan (GENIOSIL XL 10), Vinyltris(2-methoxyethoxy)silan (GENIOSIL GF 58), Vinyltriacetoxysilan.

Vorzugsweise werden als organofunktionelle Silane 3-Methacryloxypropyltrimethoxysilan (Dynasylan MEMO, Silquest A-174NT), Vinyltri(m)ethoxysilan (Dynasylan VTMO bzw. VTEO, Silquest A-151 bzw. A-171), Methyltri(m) ethoxysilan (Dynasylan MTMS bzw. MTES), beta-(3,4-Epoxycyclohexyl)ethyltrimethoxysilan (Silquest A-186), Bis (triethoxysilyl)ethan (Silquest Y-9805), gamma-Isocyanatopropyltrimethoxysilan (Silquest A-Link 35, GENIOSIL GF40), Methacryloxymethyltri(m)ethoxysilan (GENIOSIL XL 33, XL 36), (Methacryloxymethyl)(m)ethyldimethoxysilan (GENIOSIL XL 32, XL 34), 3-(Triethoxysilyl)propylbernsteinsäureanhydrid (GENIOSIL GF 20), Vinyltrimethoxysilan (GENIOSIL XL 10) und/ oder Vinyltris(2-methoxyethoxy)silan (GENIOSIL GF 58) verwendet.

Es ist aber auch möglich, andere organofunktionelle Silane auf die erfindungsgemäßen Mehrschichtperlglanzpigmente aufzubringen.

Weiterhin lassen sich, beispielsweise kommerziell von Degussa erhältliche, wässrige Vorhydrolysate einsetzen. Hierzu gehören u.a. wässriges Aminosiloxan (Dynasylan Hydrosil 1151), wässriges amino-/ alkylfunktionelles Siloxan (Dynasylan Hydrosil 2627 oder 2909), wässriges diaminofunktionelles Siloxan (Dynasylan Hydrosil 2776), wässriges, wässriges epoxyfunktionelles Siloxan (Dynasylan Hydrosil 2926), amino-/alkylfunktionelles Oligosiloxan (Dynasylan 1146), vinyl-/ alkylfunktionelles Oligosiloxan (Dynasylan 6598), oligomeres Vinylsilan (Dynasylan 6490) oder oligomeres kurzkettiges alkylfunktionelles Silan (Dynasylan 9896).

Bei einer bevorzugten Ausführungsform enthält das organofunktionelle Silangemisch neben wenigstens einem Silan ohne funktionelle Bindungsgruppe wenigstens ein aminofunktionelles Silan. Die Aminofunktion ist eine funktionelle Gruppe, die mit den meisten in Bindemitteln vorhandenen Gruppen eine oder mehrere chemische Wechselwirkungen eingehen kann. Dies kann eine kovalente Bindung, wie z.B. mit Isocyanat- oder Carboxylatfunktionen des Bindemittels, oder Wasserstoffbrückenbindungen wie mit OH- oder COOR-Funktionen oder auch ionische Wechselwirkungen beinhalten. Eine Aminofunktion ist daher für den Zweck der chemischen Anbindung des Mehrschichtperlglanzpigments an verschiedenartige Bindemittel sehr gut geeignet.

Bevorzugt werden hierzu folgende Verbindungen genommen: 3-Aminopropyltrimethoxysilan (Dynasylan AMMO; Silquest A-1110), 3-Aminopropyltriethoxysilan (Dynasylan AMEO), [3-(2-Aminoethyl)-aminopropyl]trimethoxysilan (Dynasylan DAMO, Silquest A-1120), [3-(2-Aminoethyl)-aminopropyl]triethoxysilan, triaminofunktionelles Trimethoxysilan (Silquest A-1130), Bis-(gamma-trimethoxysilylpropyl)amin (Silquest A-1170), N-Ethyl-gammaaminoisobutyltrimethoxysilan (Silquest A-Link 15), N-Phenyl-gamma-aminopropyltrimethoxysilan (Silquest Y-9669), 4-Amino-3,3-dimethylbutyltrimethoxysilan (Silquest A-1637), N-Cyclohexylaminomethylmethyldiethoxysilan (GENIOSIL XL 924), N-Cyclohexylaminomethyltriethoxysilan (GENIOSIL XL 926), N-Phenylaminomethyltrimethoxysilan (GENIOSIL XL 973) und deren Mischungen.

Bei einer weiterhin bevorzugten Ausführungsform ist das Silan ohne funktionelle Bindungsgruppe ein Alkylsilan. Das Alkylsilan weist vorzugsweise die Formel (A) auf:

$$R_{(4-z)}Si(X)_z, \qquad (A)$$

Hierbei ist z eine ganze Zahl von 1 bis 3, R ist eine substituierte oder unsubstituierte, unverzweigte oder verzweigte Alkylkette mit 10 bis 22 C-Atomen und X steht für eine Halogen- und/ oder Alkoxygruppe. Bevorzugt sind Alkylsilane mit Alkylketten mit mindestens 12 C-Atomen. R kann auch zyklisch mit Si verbunden sein, wobei in diesem Fall z üblicherweise 2 ist.

An oder auf der Oberfläche der erfindungsgemäßen Mehrschichtperlglanzpigmente können neben den erwähnten Silanen bzw. Silangemischen auch weitere organischchemische Modifizierungsmittel, wie beispielsweise substituierte oder nicht substituierte Alkylreste, Polyether, Thioether, Siloxane, etc. und Mischungen davon angeordnet sein. Es können aber auch anorganisch-chemische Modifizierungsmittel (z.B. $Al_2O_3$ oder $ZrO_2$ oder deren Gemische), welche z.B. die Dispergierbarkeit und/oder Verträglichkeit im jeweiligen Anwendungsmedium erhöhen können, auf die Pigmentoberfläche aufgebracht werden.

Über die Oberflächenmodifizierung kann beispielsweise die Hydrophilie oder Hydrophobie der Pigmentoberfläche verändert und/ oder eingestellt werden. Beispielsweise können über die Oberflächenmodifizierung die Leafing- bzw. Nonleafing-Eigenschaften der erfindungsgemäßen Mehrschichtperlglanzpigmente verändert und/ oder eingestellt werden. Unter Leafing wird verstanden, dass sich die erfindungsgemäßen Mehrschichtperlglanzpigmente in einem Anwendungsmedium, beispielsweise einem Lack oder einer Druckfarbe, an oder in der Nähe der Grenz- oder Oberfläche des Anwendungsmediums anordnen.

Die Oberflächenmodifizierungsmittel können auch reaktive chemische Gruppen, wie beispielsweise Acrylat-, Methacrylat-, Vinyl-, Isocyanat-, Cyano-, Epoxy-, Hydroxy-, Aminogruppen oder Mischungen davon, aufweisen. Diese chemisch reaktiven Gruppen ermöglichen eine chemische Anbindung, insbesondere Ausbildung von kovalenten Bindungen, an das Anwendungsmedium oder Komponenten des Anwendungsmediums, wie beispielsweise Bindemittel. Hierdurch können beispielsweise die chemischen und/ oder physikalischen Eigenschaften von ausgehärteten Lacken, Farben oder Druckfarben wie Beständigkeit gegenüber Umwelteinflüssen wie Feuchtigkeit, Sonneneinstrahlung, UV-Beständigkeit, etc., oder gegenüber mechanischen Einflüssen, beispielsweise Kratzern etc., verbessert werden.

Die chemische Reaktion zwischen den chemisch-reaktiven Gruppen und dem Anwendungsmedium bzw. Komponenten des Anwendungsmediums kann beispielsweise durch Einstrahlung von Energie, beispielsweise in Form von UV-Strahlung und/ oder Wärme, induziert werden.

Für die Einarbeitung von mit Silanen nachbeschichteten bzw. mit einer äußeren Schutzschicht versehenen Mehrschichtperlglanzpigmenten in kosmetischen Formulierungen muss darauf geachtet werden, dass das entsprechende Silan bzw. das Material der äußeren Schutzschicht nach der Kosmetikverordnung zulässig ist.

Bei einer weiteren Ausführungsform umfasst die vorliegende Erfindung Mehrschichtperlglanzpigmente, umfassend mit optisch wirksamer Beschichtung versehene plättchenförmige transparente Substrate, wobei die optisch wirksamen Beschichtung wenigstens

(a) eine nicht absorbierende hochbrechende Schicht A mit einem Brechungsindex $n \geq 1,8$,
(b) eine nicht absorbierende niedrigbrechende Schicht B mit einem Brechungsindex $n < 1,8$,
(c) eine nicht absorbierende hochbrechende Schicht C mit einem Brechungsindex $n \geq 1,8$
sowie
(d) optional wenigstens eine äußere Schutzschicht D

umfasst und die Mehrschichtperlglanzpigmente eine Summenhäufigkeitsverteilung einer volumengemittelten Größenverteilungsfunktion mit den Kennzahlen $D_{10}$, $D_{50}$, $D_{90}$ und einem Span $\Delta D$ in einem Bereich von 0,8 - 1,2 aufweisen, wobei der Span $\Delta D$ gemäß Formel (I)

$$\Delta D = (D_{90} - D_{10})/ D_{50} \qquad (I)$$

berechnet ist.

Bei einer weiteren Ausführungsform umfassen die hochbrechenden Schichten A und C jeweils Titandioxid und die niedrigbrechende Schicht B Siliziumdioxid. In einer weiteren Ausführungsform bestehen die hochbrechenden Schichten A und C jeweils aus Titandioxid und die niedrigbrechende Schicht B aus Siliziumdioxid.

In einer weiteren Ausführungsform umfasst die vorliegende Erfindung Mehrschichtperlglanzpigmente, umfassend mit optisch wirksamer Beschichtung versehene plättchenförmige transparente Substrate, wobei die optisch wirksame Beschichtung wenigstens

(a) eine nicht absorbierende hochbrechende Schicht A mit einem Brechungsindex $n \geq 1,8$,
(b) eine nicht absorbierende niedrigbrechende Schicht B mit einem Brechungsindex $n < 1,8$,
(c) eine nicht absorbierende hochbrechende Schicht C mit einem Brechungsindex $n \geq 1,8$
sowie
(d) optional wenigstens eine äußere Schutzschicht D

umfasst und die Mehrschichtperlglanzpigmente eine Summenhäufigkeitsverteilung einer volumengemittelten Größenverteilungsfunktion mit den Kennzahlen $D_{10}$, $D_{50}$, $D_{90}$ und einem Span $\Delta D$ in einem Bereich von 0,8 - 1,2 aufweisen, wobei der Span $\Delta D$ gemäß Formel (I)

$$\Delta D = (D_{90} - D_{10})/ D_{50} \qquad (I)$$

berechnet ist und die Mehrschichtperlglanzpigmente ein Chroma $C^*_{15} > 20$ besitzen.

Bei einer weiteren Ausführungsform umfasst die vorliegende Erfindung Mehrschichtperlglanzpigmente, umfassend mit optisch wirksamer Beschichtung versehene plättchenförmige transparente Substrate, wobei die optisch wirksame Beschichtung wenigstens

(a) eine nicht absorbierende hochbrechende Schicht A mit einem Brechungsindex $n \geq 1{,}8$ und einer optischen Schichtdicke von 30 nm bis 900 nm,
(b) eine nicht absorbierende niedrigbrechende Schicht B mit einem Brechungsindex $n < 1{,}8$ und einer optischen Schichtdicke > 150 nm,
(c) eine nicht absorbierende hochbrechende Schicht C mit einem Brechungsindex $n \geq 1{,}8$ und einer optischen Schichtdicke von 30 nm bis 900 nm
sowie
(d) optional wenigstens eine äußere Schutzschicht D

umfasst und die Mehrschichtperlglanzpigmente eine Summenhäufigkeitsverteilung einer volumengemittelten Größenverteilungsfunktion mit den Kennzahlen $D_{10}$, $D_{50}$, $D_{90}$ und einem Span $\Delta D$ in einem Bereich von 0,8 -1,2 aufweisen, wobei der Span $\Delta D$ gemäß Formel (I)

$$\Delta D = (D_{90} - D_{10})/ D_{50} \qquad\qquad (I)$$

berechnet ist.

In einer weiteren Ausführungsform umfasst die vorliegende Erfindung Mehrschichtperlglanzpigmente, umfassend mit optisch wirksamer Beschichtung versehene plättchenförmige transparente Substrate, wobei die optisch wirksame Beschichtung wenigstens

(a) eine nicht absorbierende hochbrechende Schicht A mit einem Brechungsindex $n \geq 1{,}8$ und einer optischen Schichtdicke von 30 nm bis 900 nm,
(b) eine nicht absorbierende niedrigbrechende Schicht B mit einem Brechungsindex $n < 1{,}8$ und einer optischen Schichtdicke $\leq 150$ nm,
(c) eine nicht absorbierende hochbrechende Schicht C mit einem Brechungsindex $n \geq 1{,}8$ und einer optischen Schichtdicke von 30 nm bis 900 nm
sowie
(d) optional wenigstens eine äußere Schutzschicht D

umfasst und die Mehrschichtperlglanzpigmente eine Summenhäufigkeitsverteilung einer volumengemittelten Größenverteilungsfunktion mit den Kennzahlen $D_{10}$, $D_{50}$, $D_{90}$ und einem Span $\Delta D$ in einem Bereich von 0,8 - 1,2 aufweisen, wobei der Span $\Delta D$ gemäß Formel (I)

$$\Delta D = (D_{90} - D_{10})/ D_{50} \qquad\qquad (I)$$

berechnet ist.

Ein Verfahren zum Herstellen der Mehrschichtperlglanzpigmente umfasst die folgenden Schritte:

(i) Größenklassieren der zu beschichtenden plättchenförmigen transparenten Substrate, so dass die zu beschichtenden plättchenförmigen transparenten Substrate eine volumengemittelte GrößenverteilunQsfunktion mit den Kennzahlen $D_{10}$, $D_{50}$, $D_{90}$ und einem Span $\Delta D$ in einem Bereich von 0,7 - 1,4 aufweisen, wobei der Span $\Delta D$ gemäß der Formel $\Delta D = (D_{90} - D_{10})/ D_{50}$ definiert ist,
(ii) Aufbringen wenigstens der Schichten A bis C auf die plättchenförmigen transparenten Substrate sowie optional wenigstens einer Schicht D,
oder
(iii) Aufbringen wenigstens der Schichten A bis C auf die plättchenförmigen transparenten Substrate sowie optional wenigstens einer Schicht D,
(iv) Größenklassieren der zu beschichtenden plättchenförmigen transparenten Substrate, so dass die zu beschichtenden plättchenförmigen transparenten Substrate eine volumengemittelte Größenverteilungsfunktion mit den Kennzahlen $D_{10}$, $D_{50}$, $D_{90}$ und einem Span $\Delta D$ in einem Bereich von 0,7 -1,4 aufweisen, wobei der Span $\Delta D$ gemäß der Formel $\Delta D = (D_{90} - D_{10})/ D_{50}$ definiert ist.

Wenn die Ausgangssubstrate zu groß sind, kann optional ein Zerkleinerungsschritt vor dem Größenklassieren durchgeführt werden.

Die Größenklassierung kann vor oder nach der Beschichtung der Substrate erfolgen. Vorteilhafterweise wird jedoch zuerst das Substrat klassiert und anschließend beschichtet. Die Größenklassierung wird durchgeführt und gegebenenfalls wiederholt, bis die Mehrschichtperlglanzpigmente die erfindungsgemäße Größenverteilung aufweisen.

Ein enger Span $\Delta D$ der Substrate kann durch geeignete Zerkleinerungs- und/ oder Klassierungsprozesse der zu beschichtenden Substrate erreicht werden. Die zu beschichtenden Substrate können beispielsweise durch Kugelmühle, Strahl- oder Rührwerkskugelmühle, Kollergang oder Dissolver zerkleinert werden. Der Span $\Delta D$ der Endfraktion kann durch geeignete Klassierung, wie beispielsweise eine mehrfache Nasssiebung, eingestellt werden. Weitere Klassierungsverfahren umfassen die Zentrifugation in Zyklonen oder eine Sedimentation aus einer Dispersion.

Die Zerkleinerungs- und Klassierprozesse können nacheinander erfolgen und gegebenenfalls miteinander kombiniert werden. So kann auf einen Zerkleinerungsvorgang ein Klassierungsvorgang folgen, dem sich ein weiterer Zerkleinerungsvorgang des Feinguts anschließt usw.

Die Metalloxidschichten werden vorzugsweise nasschemisch aufgebracht, wobei die zur Herstellung von Perlglanzpigmenten entwickelten nasschemischen Beschichtungsverfahren angewendet werden können. Bei der Nassbeschichtung werden die Substratpartikel in Wasser suspendiert und mit einem oder mehreren hydrolysierbaren Metallsalzen oder einer Wasserglaslösung bei einem für die Hydrolyse geeigneten pH-Wert versetzt, der so gewählt wird, dass die Metalloxide bzw. Metalloxidhydrate direkt auf dem zu beschichtenden Substrat aufgefällt wird, ohne dass es zu Nebenfällungen kommt. Der pH-Wert wird üblicherweise durch gleichzeitiges Zudosieren einer Base und/ oder einer Säure konstant gehalten. Anschließend werden die Pigmente abgetrennt, gewaschen und bei 50 - 150°C für 6 -18 Stunden getrocknet und gegebenenfalls 0,5 - 3 Stunden geglüht, wobei die Glühtemperatur im Hinblick auf die jeweils vorliegende Beschichtung optimiert werden kann. In der Regel liegen die Glühtemperaturen zwischen 550 und 1000°C, vorzugsweise zwischen 600 und 900°C. Falls gewünscht können die Pigmente nach Aufbringen einzelner Beschichtungen abgetrennt, getrocknet und gegebenenfalls geglüht werden, um dann zur Auffällung der weiteren Schichten wieder resuspendiert zu werden.

Die Auffällung der $SiO_2$-Schicht auf das zu beschichtende Substrat kann durch Zugabe einer Kalium- oder Natronwasserglaslösung bei einem geeigneten pH-Wert erfolgen. Alternativ kann die $SiO_2$-Schicht über Sol-Gel-Verfahren ausgehend von Alkoxysilanen, wie z.B. Tetraethoxysilan aufgebracht werden.

Die erfindungsgemäßen Mehrschichtperlglanzpigmente lassen sich auch vorteilhaft in Abmischungen mit transparenten und deckenden Weiß-, Bunt- und Schwarzpigmenten sowie weiteren Effektpigmenten verwenden.

Die erfindungsgemäßen Mehrschichtperlglanzpigmente können zur Herstellung von Pigmentpräparationen und Trockenpräparaten eingesetzt werden.

Weiterhin können die erfindungsgemäßen Mehrschichtperlglanzpigmente beispielsweise in kosmetischen Formulierungen, Kunststoffen, keramischen Materialien, Gläsern und Beschichtungszusammensetzungen wie Farben, Druckfarben, z.B. für den Offset-, Sieb-, Tief-, Flexo-, Sicherheitsdruck oder Bronzierung, Tinten, in Tonern, Lacken, z.B. Autolacken oder Pulverlacken, zur Lasermarkierung von Papier und Kunststoffen, zur Saatguteinfärbung, zur Einfärbung von Lebensmitteln oder pharmazeutischen Erzeugnissen oder zur Farbgebung von (Agrar-)Folien, Zeltplanen oder Textilien verwendet werden.

In kosmetischen Formulierungen können die erfindungsgemäßen Mehrschichtperlglanzpigmente mit nichtsilberner Interferenzfarbe mit für die jeweilige Anwendung geeigneten Roh-, Hilfs- und Wirkstoffen kombiniert werden. Die Konzentration der Mehrschichtperlglanzpigmente in der Formulierung kann zwischen 0,001 Gew.-% für Rinse-off-Produkte und 40,0 Gew.-% für Leave-on-Produkte liegen.

Die erfindungsgemäßen Mehrschichtperlglanzpigmente eignen sich insbesondere für die Anwendung in Kosmetika, wie z.B. Körperpuder, Gesichtspuder, gepresstem und losem Puder, Gesichtsmakeup, Pudercreme, Crememakeup, Emulsionsmakeup, Wachsmakeup, Foundation, Moussemakeup, Wangenrouge, Augenmakeup wie Lidschatten, Mascara, Eyeliner, flüssige Eyeliner, Augenbrauenstift, Lippenpflegestift, Lippenstift, Lip Gloss, Lip Liner, Haarstylingkompositionen wie Haarspray, Haarmousse, Haargel, Haarwachs, Haarmascara, permanente oder semipermanente Haarfarben, temporäre Haarfarben, Hautpflegekompositionen wie Lotions, Gele, Emulsionen sowie Nagellackkompositionen.

Zur Erzielung spezieller Farbeffekte können in den Kosmetikapplikationen neben den erfindungsgemäßen Mehrschichtperlglanzpigmenten weitere Farbmittel und/ oder herkömmliche Effektpigmente oder Mischungen hiervon in variablen Mengenverhältnissen eingesetzt werden. Als herkömmliche Effektpigmenten können beispielsweise handelsübliche Perlglanzpigmente auf der Basis von mit hochbrechenden Metalloxiden beschichtetem natürlichen Glimmer (wie z.B. die Produktgruppe Prestige der Fa. Eckart), BiOCl-Plättchen, TiO2-Plättchen, Perlglanzpigmente auf der Basis von mit hochbrechenden Metalloxiden beschichtetem synthetischen Glimmer oder basierend auf mit hochbrechenden Metalloxiden beschichteten Glasplättchen (wie z.B. die Produktgruppe MIRAGE der Fa. Eckart), $Al_2O_3$-, $SiO_2$- oder $TiO_2$-Plättchen verwendet werden. Außerdem können auch Metalleffektpigmente, wie z.B. die Produktgruppe Visionaire der Fa. Eckart, zugegeben werden. Die Farbmittel können aus anorganischen oder organischen Pigmenten ausgewählt sein.

Im Folgenden wird die Erfindung durch einige Beispiele näher erläutert, ohne auf diese beschränkt zu sein.

### I Herstellung der Pigmente

### A Klassierung der Substrate

### Beispiel 1: Klassierung von Glasflakes mit engem Span $\Delta D = 1,0$

Eine Suspension von 200 g Glasflakes (GF100M der Firma Glassflake Ltd.) in VE-Wasser (VE = vollentsalzt, ca. 3 Gew.-%ig) wurde über ein 100 $\mu$m Sieb klassiert und der Siebdurchgang wiederum über ein 63 $\mu$m Sieb gesiebt. Diese Siebprozedur wurde mit auf dem 63 $\mu$m Sieb erhaltenen Siebrückstand zweimal wiederholt. Es wurde eine Glasflakefraktion erhalten, die folgende Partikelgrößenverteilung aufwies (MALVERN Mastersizer 2000): $D_{10}$=50 $\mu$m, $D_{50}$=82 $\mu$m, $D_{90}$=132 $\mu$m, Span $\Delta D = 1,0$.

### Vergleichsbeispiel 1: Klassierung von Glasflakes mit breitem Span $\Delta D = 2,0$

Eine Suspension von 200 g Glasflakes (GF100M der Firma Glassflake Ltd.) in VE-Wasser (ca. 3 Gew.-%ig) wurde über ein 150 $\mu$m Sieb klassiert und der Siebdurchgang wiederum über ein 34 $\mu$m Sieb gesiebt. Diese Siebprozedur wurde mit auf dem 34 $\mu$m Sieb erhaltenen Siebrückstand zweimal wiederholt. Es wurde eine Glasflakefraktion erhalten, die folgende Partikelgrößenverteilung aufwies (MALVERN Mastersizer 2000): $D_{10}$=25 $\mu$m, $D_{50}$=88 $\mu$m, $D_{90}$=200 $\mu$m, Span $\Delta D = 1,99$.

### Beispiel 2: Klassierung von synthetischem Glimmer mit engem Span $\Delta D = 1,2$

Eine Suspension von 200 g Artificial Mica Sanbao 10-40 (Fa. Shantou F.T.Z. Sanbao Pearl Luster Mica Tech Co., Ltd. China) in VE-Wasser (ca. 3 Gew.-%ig) wurde über ein 34 $\mu$m Sieb klassiert und der Siebdurchgang wiederum über ein 20 $\mu$m Sieb gesiebt. Diese Siebprozedur wurde mit auf dem 20 $\mu$m Sieb erhaltenen Siebrückstand zweimal wiederholt. Es wurde eine Glimmerfraktion erhalten, die folgende Partikelgrößenverteilung aufwies (MALVERN Master-sizer 2000): $D_{10}$=14 $\mu$m, $D_{50}$=26 $\mu$m, $D_{90}$=45 $\mu$m, Span $\Delta D = 1,2$.

### Vergleichsbeispiel 2: Klassierung von synthetischem Glimmer mit breitem Span $\Delta D = 3,7$

1000g handelsüblicher unklassierter synthetischer Mica Artificial Mica Sanbao unclassified (Fa. Shantou F.T.Z. Sanbao Pearl Luster Mica Tech Co., Ltd. China) wurde mit 1000 mL VE-Wasser versetzt und dann in einem Laborkoller der Fa. American Cyanamid Company ca. 1 h delaminiert.

Der Kuchen wurde anschließend mit VE-Wasser zu einem Feststoffgehalt von 25 Gew.-% verdünnt und in einem Labordissolver der Fa. Pendraulik des Typs TD 200 1 h behandelt. Hierbei ist darauf zu achten, dass durch Kühlung die Temperatur der Suspension 80°C nicht übersteigt.

Anschließend wurde die Suspension mit VE-Wasser auf 3 Gew.-% Feststoffgehalt gebracht und über ein Laborsieb des Typs Separator der Fa. Sweco < 250$\mu$m gesiebt.

Die so erhaltene Micafraktion wurde dann über einen Büchner Trichter abgesaugt und der erhaltene Filterkuchen als Ausgangsmaterial für weitere Beschichtungen verwendet.

Es wurde eine Glimmerfraktion erhalten, die folgende Partikelgrößenverteilung aufwies (MALVERN Mastersizer 2000): $D_{10}$= 17,7 $\mu$m, $D_{50}$= 74,6 $\mu$m, $D_{90}$= 292,3 $\mu$m, Span $\Delta D = 3,7$.

### Beispiel 3: Klassierung von Glasflakes mit engem Span $\Delta D = 1,0$

Eine Suspension von 200 g Glasflakes (GF100M der Firma Glassflake Ltd.) in VE-Wasser (ca. 3 Gew.-%ig) wurde über ein 63 $\mu$m Sieb klassiert und der Siebdurchgang wiederum über ein 34 $\mu$m Sieb gesiebt. Diese Siebprozedur wurde mit auf dem 34 $\mu$m Sieb erhaltenen Siebrückstand zweimal wiederholt. Es wurde eine Glasflakefraktion erhalten, die folgende Partikelgrößenverteilung aufwies (MALVERN Mastersizer 2000): $D_{10}$=32 $\mu$m, $D_{50}$= 62 $\mu$m, $D_{90}$=93 $\mu$m, Span $\Delta D = 1,0$.

**B Herstellung einschichtiger Pigmente (Ausgangsmaterial für mehrschichtige Perlglanzpigmente)**

**Vergleichsbeispiel 3 : Herstellung des Ausgangsmaterials für das Beispiel 4**

200 g Glasflakes aus Beispiel 1 wurden in 2000 ml VE-Wasser suspendiert und unter turbulentem Rühren auf 80°C erhitzt. Der pH-Wert der Suspension wurde mit verdünnter HCl auf pH 1,9 eingestellt und sodann eine erste Schicht "SnO$_2$" auf den Glasflakes gefällt. Diese Schicht wurde durch Zugabe von einer Lösung, bestehend aus 3 g SnCl$_4$ x 5 H$_2$O (in 10 ml konz. HCl plus 50 ml VE-Wasser), unter gleichzeitiger Zudosierung von einer 10 Gew.-%igen NaOH-Lösung, um den pH-Wert konstant zu halten, über den Zeitraum von 1 h gebildet. Um die Fällung zu vervollständigen wurde die Suspension noch weitere 15 min gerührt. Danach wurde der pH-Wert mit verdünnter HCl auf pH 1,6 heruntergesetzt und sodann eine Lösung von 75 ml TiCl$_4$ (200 g TiO$_2$/l VE-Wasser) in die Suspension hinzudosiert. Der pH-Wert wurde dabei durch Gegensteuern mit 10 Gew.-%iger NaOH-Lösung konstant auf pH 1,6 gehalten. Danach wurde noch 15 min nachgerührt, abfiltriert und der Filterkuchen mit VE-Wasser nachgewaschen. Der Filterkuchen wurde bei 100°C vorgetrocknet und bei 650°C 30 min kalziniert. Es wurde ein glänzendes Effektpigment mit silberner Interferenzfarbe erhalten.

**Vergleichsbeispiel 4 : Herstellung des Ausgangsmaterials für das Beispiel 5**

200 g Glasflakes aus Beispiel 3 wurden in 2000 ml VE-Wasser suspendiert und unter turbulentem Rühren auf 80°C erhitzt. Der pH-Wert der Suspension wurde mit verdünnter HCl auf pH 1,9 eingestellt und sodann eine erste Schicht "SnO$_2$" auf den Glasflakes gefällt. Diese Schicht wurde durch Zugabe von einer Lösung, bestehend aus 3 g SnCl$_4$ x 5 H$_2$O (in 10 ml konz. HCl plus 50 ml VE-Wasser), unter gleichzeitiger Zudosierung von einer 10 Gew.-%igen NaOH-Lösung, um den pH-Wert konstant zu halten, über den Zeitraum von 1 h gebildet. Um die Fällung zu vervollständigen wurde die Suspension noch weitere 15 min gerührt. Danach wurde der pH-Wert mit verdünnter HCl auf pH 1,6 heruntergesetzt und sodann eine Lösung von 77 ml TiCl$_4$ (200 g TiO$_2$/l VE-Wasser) in die Suspension hinzudosiert. Der pH-Wert wurde dabei durch Gegensteuern mit 10 Gew.-%iger NaOH-Lösung konstant auf pH 1,6 gehalten. Danach wurde noch 15 min nachgerührt, abfiltriert und der Filterkuchen mit VE-Wasser nachgewaschen. Der Filterkuchen wurde bei 100°C vorgetrocknet und bei 650°C 30 min kalziniert. Es wurde ein glänzendes Effektpigment mit silberner Interferenzfarbe erhalten.

**Vergleichsbeispiel 5: synthetischer Glimmer/ TiO$_2$(rutil)**

200g synthetischer Glimmer aus Beispiel 2 wurden in 2000 ml VE-Wasser suspendiert und unter turbulentem Rühren auf 80°C erhitzt. Der pH-Wert der Suspension wurde mit verdünnter HCl auf pH 1,9 eingestellt und sodann eine erste Schicht "SnO$_2$" auf den synthetischen Glimmer gefällt. Diese Schicht wurde durch Zugabe einer Lösung, bestehend aus 5 g SnCl$_4$ x 5 H$_2$O (in 10 ml konz. HCl plus 50 ml VE-Wasser), unter gleichzeitiger Zudosierung einer 10 Gew.-%igen NaOH-Lösung, um den pH-Wert konstant zu halten, über den Zeitraum von 1 h gebildet. Um die Fällung zu vervollständigen wurde die Suspension noch weitere 15 min gerührt. Danach wurde der pH-Wert mit verdünnter HCl auf pH 1,6 heruntergesetzt und sodann eine Lösung von 900 ml TiCl$_4$ (200 g TiO$_2$/ l VE-Wasser) in die Suspension hinzudosiert. Der pH-Wert wurde dabei durch Gegensteuern mit 10 Gew.-%iger NaOH-Lösung konstant auf pH 1,6 gehalten. Danach wurde noch 15 min nachgerührt, abfiltriert und der Filterkuchen mit VE-Wasser nachgewaschen. Der Filterkuchen wurde bei 100°C vorgetrocknet und bei 750°C 30 min kalziniert. Es wurde ein glänzendes Perlglanzpigment mit blauer Interferenzfarbe erhalten.

**Vergleichsbeispiel 6: Herstellung des Ausgangsmaterials für das Vergleichsbeispiel 7**

200 g Glasflakes aus Vergleichsbeispiel 1 wurden in 2000 ml VE-Wasser suspendiert und unter turbulentem Rühren auf 80°C erhitzt. Der pH-Wert der Suspension wurde mit verdünnter HCl auf pH 1,9 eingestellt und sodann eine erste Schicht "SnO$_2$" auf den Glasflakes gefällt. Diese Schicht wurde durch Zugabe von einer Lösung, bestehend aus 3 g SnCl$_4$x 5 H$_2$O (in 10 ml konz. HCl plus 50 ml VE-Wasser), unter gleichzeitiger Zudosierung von einer 10 Gew.-%igen NaOH-Lösung, um den pH-Wert konstant zu halten, über den Zeitraum von 1 h gebildet. Um die Fällung zu vervollständigen wurde die Suspension noch weitere 15 min gerührt. Danach wurde der pH-Wert mit verdünnter HCl auf pH 1,6 heruntergesetzt und sodann eine Lösung von 75 ml TiCl$_4$ (200 g TiO$_2$/l VE-Wasser) in die Suspension hinzudosiert. Der pH-Wert wurde dabei durch Gegensteuern mit 10 Gew.-%iger NaOH-Lösung konstant auf pH 1,6 gehalten. Danach wurde noch 15 min nachgerührt, abfiltriert und der Filterkuchen mit VE-Wasser nachgewaschen. Der Filterkuchen wurde bei 100°C vorgetrocknet und bei 650°C 30 min kalziniert. Es wurde ein glänzendes Effektpigment mit silberner Interferenzfarbe erhalten.

**C Herstellung der Mehrschichtperlglanzpigmente**

**Beispiel 4: Glasflakes/ TiO$_2$(rutil)/ SiO$_2$/ TiO$_2$(rutil)**

200 g TiO$_2$-beschichtete Glasflakes aus Vergleichsbeispiel 3 wurden in 1300 ml VE-Wasser suspendiert und unter turbulentem Rühren auf 80°C erhitzt. Der pH-Wert wurde mit 5 Gew.-%iger NaOH-Lösung auf 7,5 angehoben und 15 min gerührt. Eine Wasserglaslösung (255 g Wasserglaslösung, 27 Gew.-% SiO$_2$, gemischt mit 255 g VE-Wasser) wurde sodann langsam in die Suspension eingeleitet und der pH-Wert konstant bei pH 7,5 gehalten. Danach wurde noch 20 min nachgerührt und der pH-Wert auf 1,9 gesenkt. Dann schied man eine Schicht "SnO$_2$" auf der SiO$_2$-Oberfläche ab. Diese Schicht wurde durch Zugabe von einer Lösung, bestehend aus 3 g SnCl$_4$ x 5 H$_2$O (in 10 ml konz. HCl plus 50 ml VE-Wasser), unter gleichzeitiger Zudosierung von einer 10 Gew.-%igen NaOH-Lösung, um den pH-Wert konstant zu halten, über den Zeitraum von 1 h gebildet. Um die Fällung zu vervollständigen wurde die Suspension noch weitere 15 min gerührt. Danach wurde der pH-Wert mit verdünnter HCl auf pH 1,6 heruntergesetzt und sodann eine Lösung von 95 ml TiCl$_4$ (200 g TiO$_2$/l VE-Wasser) in die Suspension hinzudosiert. Der pH-Wert wurde dabei durch Gegensteuern mit 10 Gew.-%iger NaOH-Lösung konstant auf pH 1,6 gehalten. Danach wurde noch 15 min nachgerührt, abfiltriert und der Filterkuchen mit VE-Wasser nachgewaschen. Der Filterkuchen wurde bei 100°C vorgetrocknet und bei 650°C 30 min kalziniert. Es wurde ein extrem hochglänzendes Mehrschichtperlglanzpigmente mit roter Interferenzfarbe im Glanzwinkel erhalten, die im flachen Beobachtungswinkel zu einem grünlichen Goldton abkippt.

**Vergleichsbeispiel 7: Glasflakes/ TiO$_2$(rutil)/ SiO$_2$/ TiO$_2$(rutil)**

200 g TiO$_2$-beschichtete Glasflakes aus Vergleichsbeispiel 6 wurden in 1300 ml VE-Wasser suspendiert und unter turbulentem Rühren auf 80°C erhitzt. Der pH-Wert wurde mit 5 Gew.-%iger NaOH-Lösung auf 7,5 angehoben und 15 min gerührt. Eine Wasserglaslösung (255 g Wasserglaslösung, 27 Gew.-% SiO$_2$, gemischt mit 255 g VE-Wasser) wurde sodann langsam in die Suspension eingeleitet und der pH-Wert konstant bei pH 7,5 gehalten. Danach wurde noch 20 min nachgerührt und der pH-Wert auf 1,9 gesenkt. Dann schied man eine Schicht "SnO$_2$" auf der SiO$_2$-Oberfläche ab. Diese Schicht wurde durch Zugabe von einer Lösung, bestehend aus 3 g SnCl$_4$ x 5 H$_2$O (in 10 ml konz. HCl plus 50 ml VE-Wasser), unter gleichzeitiger Zudosierung von einer 10 Gew.-%igen NaOH-Lösung, um den pH-Wert konstant zu halten, über den Zeitraum von 1 h gebildet. Um die Fällung zu vervollständigen wurde die Suspension noch weitere 15 min gerührt. Danach wurde der pH-Wert mit verdünnter HCl auf pH 1,6 heruntergesetzt und sodann eine Lösung von 95 ml TiCl$_4$ (200 g TiO$_2$/l VE-Wasser) in die Suspension hinzudosiert. Der pH-Wert wurde dabei durch Gegensteuern mit 10 Gew.-%iger NaOH-Lösung konstant auf pH 1,6 gehalten. Danach wurde noch 15 min nachgerührt, abfiltriert und der Filterkuchen mit VE-Wasser nachgewaschen. Der Filterkuchen wurde bei 100°C vorgetrocknet und bei 650°C 30 min kalziniert. Es wurde ein Mehrschichtperlganzpigment mit rötlich-blauer Interferenzfarbe erhalten.

**Vergleichsbeispiel 8: synthetischer Glimmer/ TiO$_2$(rutil)/ SiO$_2$/ TiO$_2$(rutil)**

200 g synthetischer Glimmer aus Vergleichsbeispiel 2 mit einem Span von 3,7 wurden in 2000 ml VE-Wasser suspendiert und unter turbulentem Rühren auf 80°C erhitzt. Der pH-Wert der Suspension wurde mit verdünnter HCl auf pH 1,9 eingestellt und sodann eine erste Schicht "SnO$_2$" auf den synthetischen Glimmer gefällt. Diese Schicht wurde durch Zugabe einer Lösung, bestehend aus 6 g SnCl$_4$ x 5 H$_2$O (in 10 ml konz. HCl plus 50 ml VE-Wasser), unter gleichzeitiger Zudosierung einer 10 Gew.-%igen NaOH-Lösung, um den pH-Wert konstant zu halten, über den Zeitraum von 1 h gebildet. Um die Fällung zu vervollständigen wurde die Suspension noch weitere 15 min gerührt. Danach wurde der pH-Wert mit verdünnter HCl auf pH 1,6 heruntergesetzt und sodann eine Lösung von 320 ml TiCl$_4$ (200 g TiO$_2$/l VE-Wasser) in die Suspension hinzudosiert. Der pH-Wert wurde dabei durch Gegensteuern mit 10 Gew.-%iger NaOH-Lösung konstant auf pH 1,6 gehalten. Danach wurde der pH-Wert mit 5 Gew.-%iger NaOH-Lösung auf 7,5 angehoben und 15 min gerührt. Eine Wasserglaslösung (200 g Wasserglaslösung, 24 Gew.-% SiO$_2$, gemischt mit 207 g VE-Wasser) wurde sodann langsam in die Suspension eingeleitet und der pH-Wert konstant bei pH 7,5 gehalten. Danach wurde noch 20 min nachgerührt und der pH-Wert wieder auf 1,9 gesenkt. Dann schied man eine zweite Schicht "SnO$_2$" auf der SiO$_2$-Oberfläche ab. Diese Schicht wurde durch Zugabe von einer Lösung, bestehend aus 6 g SnCl$_4$ x 5 H$_2$O (in 10 ml konz. HCl plus 50 ml VE-Wasser), unter gleichzeitiger Zudosierung von einer 10 Gew.-%igen NaOH-Lösung, um den pH-Wert konstant zu halten, über den Zeitraum von 1 h gebildet. Um die Fällung zu vervollständigen wurde die Suspension noch weitere 15 min gerührt. Danach wurde der pH-Wert mit verdünnter HCl auf pH 1,6 heruntergesetzt und sodann eine Lösung von 300 ml TiCl$_4$ (200 g TiO$_2$/ l VE-Wasser) in die Suspension hinzudosiert. Der pH-Wert wurde dabei durch Gegensteuern mit 10 Gew.-%iger NaOH-Lösung konstant auf pH 1,6 gehalten. Danach wurde noch 15 min nachgerührt, abfiltriert und der Filterkuchen mit VE-Wasser nachgewaschen. Der Filterkuchen wurde bei 100°C vorgetrocknet und bei 750°C 30 min kalziniert. Es wurde ein schwach glänzendes Mehrschichtperlglanzpigment mit blauer Interferenzfarbe und einem Span von 3,7 erhalten.

**Beispiel 5: Glasflakes/ TiO$_2$(rutil)i SiO$_2$/ TiO$_2$(rutil)**

200 g TiO$_2$-beschichtete Glasflakes aus Vergleichsbeispiel 4 wurden in 1300 ml VE-Wasser suspendiert und unter turbulentem Rühren auf 80°C erhitzt. Der pH-Wert wurde mit 5 Gew.-%iger NaOH-Lösung auf 7,5 angehoben und 15 min gerührt. Eine Wasserglaslösung (40 g Wasserglaslösung, 27 Gew.-% SiO$_2$, gemischt mit 40 g VE-Wasser) wurde sodann langsam in die Suspension eingeleitet und der pH-Wert konstant bei pH 7,5 gehalten. Danach wurde noch 20 min nachgerührt und der pH-Wert auf 1,9 gesenkt. Dann schied man eine Schicht "SnO$_2$" auf der SiO$_2$-Oberfläche ab. Diese Schicht wurde durch Zugabe von einer Lösung, bestehend aus 3 g SnCl$_4$ x 5 H$_2$O (in 10 ml konz. HCl plus 50 ml VE-Wasser), unter gleichzeitiger Zudosierung von einer 10 Gew.-%igen NaOH-Lösung, um den pH-Wert konstant zu halten, über den Zeitraum von 1 h gebildet. Um die Fällung zu vervollständigen wurde die Suspension noch weitere 15 min gerührt. Danach wurde der pH-Wert mit verdünnter HCl auf pH 1,6 heruntergesetzt und sodann eine Lösung von 200 ml TiCl$_4$ (200 g TiO$_2$/l VE-Wasser) in die Suspension hinzudosiert. Der pH-Wert wurde dabei durch Gegensteuern mit 10 Gew.-%iger NaOH-Lösung konstant auf pH 1,6 gehalten. Danach wurde noch 15 min nachgerührt, abfiltriert und der Filterkuchen mit VE-Wasser nachgewaschen. Der Filterkuchen wurde bei 100°C vorgetrocknet und bei 650°C 30 min kalziniert. Es wurde ein extrem hochglänzendes Mehrschichtperlglanzpigmente mit goldener Interferenzfarbe im erhalten.

**Beispiel 6: synthetischer Glimmer/ TiO$_2$(rutil)/ SiO$_2$/ TiO$_2$(rutil)**

200g synthetischer Glimmer aus Beispiel 2 mit einem Span von 1,2 wurden in 2000 ml VE-Wasser suspendiert und unter turbulentem Rühren auf 80°C erhitzt. Der pH-Wert der Suspension wurde mit verdünnter HCl auf pH 1,9 eingestellt und sodann eine erste Schicht "SnO$_2$" auf den synthetischen Glimmer gefällt. Diese Schicht wurde durch Zugabe einer Lösung, bestehend aus 6 g SnCl$_4$ x 5 H$_2$O (in 10 ml konz. HCl plus 50 ml VE-Wasser), unter gleichzeitiger Zudosierung einer 10 Gew.-%igen NaOH-Lösung, um den pH-Wert konstant zu halten, über den Zeitraum von 1 h gebildet. Um die Fällung zu vervollständigen wurde die Suspension noch weitere 15 min gerührt. Danach wurde der pH-Wert mit verdünnter HCl auf pH 1,6 heruntergesetzt und sodann eine Lösung von 320 ml TiCl$_4$ (200 g TiO$_2$/ VE-Wasser) in die Suspension hinzudosiert. Der pH-Wert wurde dabei durch Gegensteuern mit 10 Gew.-%iger NaOH-Lösung konstant auf pH 1,6 gehalten. Danach wurde der pH-Wert mit 5 Gew.-%iger NaOH-Lösung auf 7,5 angehoben und 15 min gerührt. Eine Wasserglaslösung (200 g Wasserglaslösung, 24 Gew.-% SiO$_2$, gemischt mit 207 g VE-Wasser) wurde sodann langsam in die Suspension eingeleitet und der pH-Wert konstant bei pH 7,5 gehalten. Danach wurde noch 20 min nachgerührt und der pH-Wert wieder auf 1,9 gesenkt. Dann schied man eine zweite Schicht "SnO$_2$" auf der SiO$_2$-Oberfläche ab. Diese Schicht wurde durch Zugabe von einer Lösung, bestehend aus 6 g SnCl$_4$ x 5 H$_2$O (in 10 ml konz. HCl plus 50 ml VE-Wasser), unter gleichzeitiger Zudosierung von einer 10 Gew.-%igen NaOH-Lösung, um den pH-Wert konstant zu halten, über den Zeitraum von 1 h gebildet. Um die Fällung zu vervollständigen wurde die Suspension noch weitere 15 min gerührt. Danach wurde der pH-Wert mit verdünnter HCl auf pH 1,6 heruntergesetzt und sodann eine Lösung von 100 ml TiCl$_4$ (200 g TiO$_2$/l VE-Wasser) in die Suspension hinzudosiert. Der pH-Wert wurde dabei durch Gegensteuern mit 10 Gew.-%iger NaOH-Lösung konstant auf pH 1,6 gehalten. Danach wurde noch 15 min nachgerührt, abfiltriert und der Filterkuchen mit VE-Wasser nachgewaschen. Der Filterkuchen wurde bei 100°C vorgetrocknet und bei 750°C 30 min kalziniert. Es wurde ein sehr brillantes Mehrschichtperlglanzpigment mit extrem blauer Interferenzfarbe erhalten.

## II Physikalische Charakterisierung

### IIa Winkelabhängige Farbmessungen

Zur Messung der Chromawerte wurden die Mehrschichtperlglanzpigmente mit einer Pigmentierungshöhe von 6 Gew.-% (bezogen auf das Gesamtgewichtes des Nasslacks) in einen konventionellen Nitrocelluloselack (Dr. Renger Erco Bronzemischlack 2615e; Fa. Morton) eingerührt. Dabei wurden die Mehrschichtperlglanzpigmente vorgelegt und anschließend mit einem Pinsel in den Lack dispergiert.

Der fertige Lack wurde auf einem Rakelabzugsgerät (RK Print Coat Instr. LTd. Citenco Abziehgerät Modell K 101) mit einer Nassfilmdicke je nach D$_{50}$-Wert des Mehrschichtperlglanzpigments gemäß Tabelle 1 auf Byk-Gardner Schwarz-Weiß-Rakelkarten (Byko-Chart 2853) appliziert und nachfolgend bei Raumtemperatur getrocknet.

Mit einem Mehrwinkelfarbmessgerät, Byk Mac (Fa. Byk Gardener) wurden bei einem konstanten Einfallswinkel von 45° (gemäß Herstellerangaben) bei verschiedenen Beobachtungswinkeln relativ zum Glanzwinkel die L*- und C*-Werte bestimmt. Insbesondere waren die Beobachtungswinkel relativ nahe am Glanzwinkel bei 15° sowie -15° relevant. Als relevanter Chromawert der erfindungsgemäßen Mehrschichtperlglanzpigmente wurde der C*$_{15}$-Wert herangezogen, welcher bei einem 15° vom Glanz entfernten Winkel gemessen wurde.

Stark reflektierende Proben (Idealfall Spiegel) reflektierten nahezu das gesamte eintreffende Licht im sogenannten Glanzwinkel. Hier erschien der Farbeindruck der Interferenzfarbe am stärksten. Je weiter man sich bei der Messung vom Glanzwinkel entfernte, desto weniger Licht und somit Interferenzeffekt konnte gemessen werden.

**IIb Glanzmessungen**

Der Glanz ist ein Maß für die gerichtete Reflexion und lässt sich mittels eines Micro-Tri-Gloss-Gerätes charakterisieren. Stärker streuende Proben weisen mithin einen niedrigen Glanz auf.

Es wurden die Nitrolackapplikationen aus IIa mit Hilfe eines Micro-Tri-Gloss Glanzmessgerätes der Fa. Byk Gardner bei einem Messwinkel von 20° für hochglänzende Proben und bei 60° für mittelglänzende Proben auf schwarzem Hintergrund vermessen. Lagen die Glanzwerte bei 60° über 70 Glanzeinheiten, so werden die Proben bei 20° gemessen (Byk-Gardner Katalog 2007/ 2008, S. 14).

**IIc Partikelgrößenbestimmung:**

Die Größenverteilungskurve wurde mit einem Gerät der Fa. Malvern (Gerät: MALVERN Mastersizer 2000) gemäß Herstellerangaben bestimmt. Hierzu wurden ca. 0,1g des entsprechenden Pigmentes als wässrige Suspension, ohne Zusatz von Dispergierhilfsmitteln, unter ständigem Rühren mittels einer Pasteurpipette in die Probenvorbereitungszelle des Messgerätes gegeben und mehrmals vermessen. Aus den einzelnen Messergebnissen wurden die resultierenden Mittelwerte gebildet. Die Auswertung der Streulichtsignale erfolgte dabei nach der Mie-Theorie, welche auch Brechungs- und Absorptionsverhalten der Partikel beinhaltet (Abbildung 2).

Unter der mittleren Größe $D_{50}$ wird im Rahmen dieser Erfindung der $D_{50}$-Wert der Summendurchgangskurve der Volumengemittelten Größenverteilungsfunktion, wie sie durch Laserbeugungsmethoden erhalten werden, verstanden. Der $D_{50}$-Wert gibt an, dass 50 % der Pigmente einen Durchmesser aufweisen, der gleich oder kleiner als der angegebene Wert, beispielsweise 20 $\mu$m, ist.

Entsprechend gibt der $D_{90}$-Wert an, dass 90% der Pigmente einen Durchmesser aufweisen, der gleich oder kleiner des jeweiligen Wertes ist.

Weiterhin gibt der $D_{10}$-Wert an, dass 10% der Pigmente einen Durchmesser aufweisen, der gleich oder kleiner des jeweiligen Wertes ist.

Der Span $\Delta D$, definiert als $\Delta D = (D_{90} - D_{10})/D_{50}$, gibt die Verteilungsbreite wieder.

**III Ergebnisse**

Tabelle 3: Charakterisierung der Effektpigmente

| Effektpigment | Aufbau | Glanz, 20° | $C^*_{15}$ | Span |
|---|---|---|---|---|
| Vergleichsbeispiel 3 | Glasflake/$TiO_2$ | 78,0 | 6,0 | 1,1 |
| Vergleichsbeispiel 4 | Glasflake/$TiO_2$ | 44,0 | 5,7 | 1,1 |
| Vergleichsbeispiel 6 | Glasflake/$TiO_2$ | 67,8 | 6,4 | 2,0 |
| Beispiel 4 | Glasflake/$TiO_2$/$SiO_2$/$TiO_2$ | 95,4 | 24,4 | 1,1 |
| Vergleichsbeispiel 7 | Glasflake/$TiO_2$/$SiO_2$/$TiO_2$ | 70,5 | 14,6 | 2,0 |
| Beispiel 5 | Glasflake/$TiO_2$/$SiO_2$/$TiO_2$ | 53,7 | 21,3 | 1,1 |

Anhand der Daten in Tabelle 3 kann man eindeutig erkennen, dass das erfindungsgemäße Beispiel 4 mit dem Schichtaufbau Glasflake/$TiO_2$/$SiO_2$I$TiO_2$ und geringem Span mit 17.4 Einheiten einen starken Glanzgewinn im Vergleich zum Ausgangsmaterial aufwies. Für das Vergleichsbeispiel 7 mit großem Span fand man lediglich einen um 2.7 Einheiten erhöhten Glanzwert. Auch das Chroma des erfindungsgemäßen Beispiels 4 zeigt sich mit 24,4 Einheiten zum Vergleichsbeispiel 7 mit 14.6 Einheiten signifikant erhöht.

Für das erfindungsgemäße Beispiel 5 findet im Vergleich zum Ausgangsmaterial Vergleichsbeispiel 4 einen um 9.7 Einheiten erhöhten Glanzwert.

Tabelle 4: Charakterisierung der Effektpigmente

| Effektpigment | Aufbau | Glanz, 60° | $C^*_{15}$ | Span |
|---|---|---|---|---|
| Beispiel 6 | synth. Glimmer/ $TiO_2$/ $SiO_2$/ $TiO_2$ | 67,9 | 29,1 | 1,2 |
| Vergleichsbeispiel 5 | synth. Glimmer/ $TiO_2$ | 29,5 | 25,6 | 1,2 |
| Vergleichsbeispiel 8 | synth. Glimmer/ $TiO_2$/ $SiO_2$/ $TiO_2$ | 19,1 | 16,5 | 3,7 |

Laut Tabelle 4 ist ein extremer Glanzsteigerungseffekt bei dem erfindungsgemäßen Beispiel 6 im Vergleich zu den Vergleichsbeispielen 5 und 8 zu beobachten. Die Kombination aus engem Span mit der Mehrschichttechnologie weist deutliche Vorteile zu den Einzeltechnologien auf.

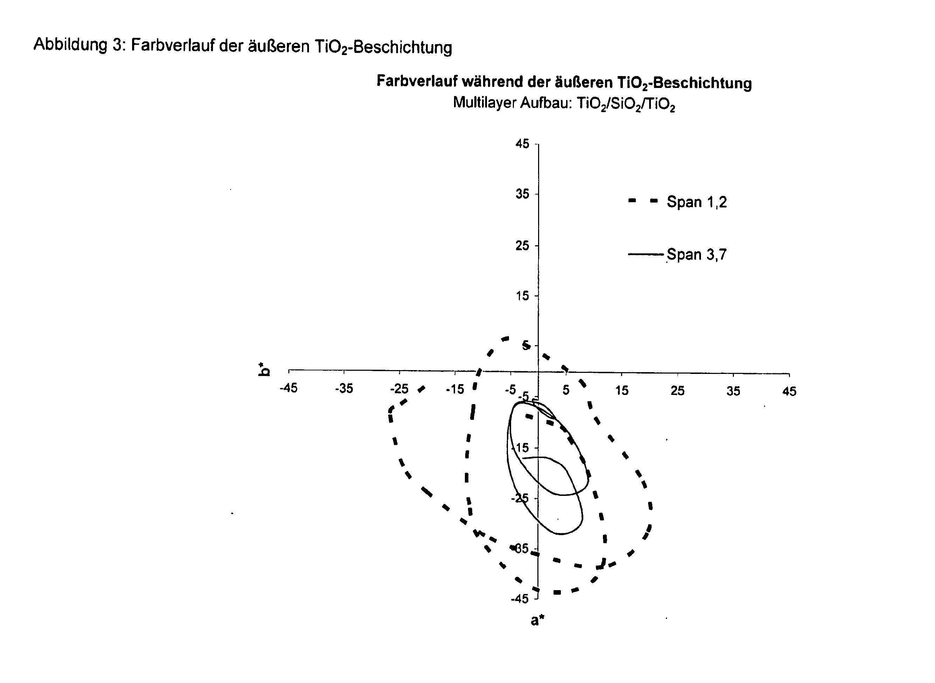

In Abbildung 3 kann man signifikant erkennen, dass ein engerer Span nicht nur eine deutliche Glanzsteigerung, sondern auch eine deutliche Erhöhung der Chromawerte von mehrschichtigen Perlglanzeffektpigmenten bedingt. In Abbildung 3 ist der Farbverlauf bei zunehmender Titandioxidschichtdicke auf einem Substrat mit dem Aufbau synthetischer Glimmer/$TiO_2$ (ca. 50nm)/$SiO_2$ (ca. 40nm) mit einem Span von 1,2 im Vergleich zum Farbverlauf mit einem Substrat gleichen Aufbaus jedoch mit einem Span von 3,7 zu beobachten.

## IV. Anwendungstechnische Beispiele

In den nachfolgenden kosmetischen Anwendungsbeispielen wurden die erfindungsgemäßen Mehrschichtperlglanzpigmente verwendet, die nach einem der vorstehenden Beispiele hergestellt wurden.

**Beispiel 7:** Transparenter Lippenstift

| INCI Name | Produktname | Gew-% | Lieferant |
|---|---|---|---|
| *Phase A* | | *100.00* | |
| Ethylenediamine/Hydrogenated Dimer Dilinoleate Copolymer Bis-Di-C14-18 Alkyl Amide | Sylvaclear A2614V | 28.00 | www.arizonachemical.com |
| Bis-Stearyl Ethylenediamine/ Neopentyl Glycol/Hydrogenated Dimer Dilinoleate | Sylvaclear C75V | 28.00 | www.arizonachemical.com |
| Paraffinum Liquidum | Paraffinum Liquidum | 13.80 | www.heess.de |
| Macadamia Integrifolia Seed Oil | Floramac Hawaiian Macadamia Oil-Refined | 10.00 | www.floratech.com |
| Isopropyl Myristate | Isopropyl Myristate | 6.00 | www.vwr.com |
| C12-15 Alkyl Benzoate | Sympatens-LBZ | 6.00 | www. kolb.ch |
| Caprylic/ Capric Triglyceride | Miglyol 812 | 7.00 | www.sasolwax.com |
| Propylparaben | Propyl-4-hydroxybenzoat | 0.20 | www.sigmaaldrich.com |
| *Phase B* | | | |
| **Mehrschicht-perlglanzpigment** | **Mehrschicht-perlglanzpigment** | **1.00** | |

Das Mehrschichtperlglanzpigment kann in einem Bereich von 0,01 - 5,0 Gew.-% eingesetzt werden. Der Ausgleich kann mit Paraffinum Liquidum erfolgen.

Phase A wurde auf 85°C erhitzt, danach wurde Phase B der Phase A hinzugegeben und gemischt. Anschließend wurde die Mischung mit einer Temperatur von 75°C in einem Lippenstiftform abgefüllt.

**Beispiel 8:** Body Lotion Water-in-Silicone

| INCI Name | Produktname | Gew-% | Lieferant |
|---|---|---|---|
| *Phase A* | | *100.00* | |
| Cyclopentasiloxane (and) Dimethiconol | Dow Corning 1501 | 11.20 | www.dowcorning.com |
| Cyclopentasiloxane | Dow Corning 245 | 5.75 | www.dowcorning.com |
| Cyclopentasiloxane (and) PEG/PPG/-18/18 Dimethicone | Dow Corning 5225 C | 13.80 | www.dowcorning.com |
| C 30-45 Alkyl Methicone | Dow Corning Cosmetic Wax AMS-C30 | 3.45 | www.dowcorning.com |
| **Mehrschicht-perlglanzpigment** | **Mehrschicht-perlglanzpigment** | **1.50** | |
| *Phase B* | | | |
| Polysorbate 20 | Tween 20 | 0.60 | www.uniqema.com |
| Phenoxyethanol (and) Methylparaben (and) Ethylparaben (and) Butylparaben | Uniphen P-23 | 0.35 | www.induchem.com |
| Sodium Chloride | Sodium Chloride | 0.75 | www.vwr.com |
| Aqua | Wasser | 62.60 | |

Das Mehrschichtperlglanzpigment kann in einem Bereich von 0,5 - 2,5 Gew.-% eingesetzt werden. Der Ausgleich kann mit Wasser erfolgen.

Phase A wurde gemischt und auf 75°C erhitzt, Phase B nach dem Mischen auf 70°C erhitzt, anschließend wurde Phase B langsam unter Homogenisierung zu Phase A hinzugefügt. Unter Rühren wurde die Emulsion abgekühlt und in ein angemessenes Behältnis abgefüllt.

**Beispiel 9**: Duschgel

| INCI Name | Produktname | Gew-% | Lieferant |
|---|---|---|---|
| *Phase A* | | *100.00* | |
| **Mehrschichtperl-glanzpigment** | **Mehrschichtperl-glanzpigment** | **0.01** | |
| Aqua | Wasser | 68.21 | |
| Blue 1 (0.5 Gew.-% aqueous solution) | Blue 1 | 0.10 | www.sunchemicals.com |
| Acrylates/C10-30 Alkyl Acrylate Crosspolymer | Carbopol ETD 2020 | 1.00 | www.noveon.com |
| Propylene Glycol | 1,2-Propanediol | 1.00 | www.vwr.com |
| *Phase B* | | | |
| TEA- Lauryl Sulfate | Texapon T 42 | 22.00 | www.cognis.com |
| Cocamide DEA | Rewomid DC 212 S | 3.00 | www.degussa.com |
| Cocamidopropyl Betaine | Tego Betain F 50 | 4.00 | www.cognis.com |
| Disodium EDTA | Edeta BD | 0.05 | www.basf.com |
| *Phase C* | | | |
| Triethanolamine | Triethanolamine | 0.30 | www.vwr.com |

(fortgesetzt)

| Phase C | | | |
|---|---|---|---|
| Phenoxyethanol, Ethylhexylglycerin | Euxyl PE 9010 | 0.60 | www.schuelke.com |

Das Mehrschichtperlglanzpigment kann in einem Bereich von 0,01 - 1,0 Gew.-% eingesetzt werden. Der Ausgleich kann mit Wasser erfolgen.

Carbopol wurde in Phase A dispergiert und 15 Minuten gerührt und auf 65°C erhitzt. Danach wurden die Rohstoffe der Phase B einzeln zu Phase A unter langsamem Rühren hinzugefügt. Anschließend wurde die Mischung auf 40°C abgekühlt und Phase C zugegeben.

**Beispiel 10**: Haargel

| INCI Name | Produktname | Gew-% | Lieferant |
|---|---|---|---|
| *Phase A* | | 100.00 | |
| **Mehrschichtperlglanzpigment** | **Mehrschicht-perlglanzpigment** | **0.10** | |
| Ammonium Acryloyldimehtyltaurate/ VP Copolymer | Aristoflex AVC | 1.40 | www.clariant.com |
| Citric Acid | Citric Acid | 0.10 | www.vwr.com |
| Aqua | Wasser | 55.10 | |
| *Phase B* | | | |
| PVP | Luviskol K 30 Powder | 1.50 | www.basf.com |
| Propylene Glycol, Diazolidinyl, Urea, Methylparaben, Propylparaben | Germaben II | 0.20 | www.ispcorp.com |
| Triethanolamine | Triethanolamine | 1.20 | www.vwr.com |
| Water | Aqua | 40.40 | |

Das Mehrschichtperlglanzpigment kann in einem Bereich von 0,01 - 0,5 Gew.-% eingesetzt werden. Der Ausgleich kann mit Wasser erfolgen.

Das Pigment wurde mit dem Wasser von Phase A verrührt , Aristoflex AVP und Citric Acid wurde unter Rühren hinzugegeben und bei einer Geschwindigkeit von 800 rpm für 15 Minuten gemischt. Die Inhaltsstoffe von Phase B wurden aufgelöst bis eine homogene Lösung entstand, anschließend wurde Phase B zu Phase A gegeben und gemischt.

**Beispiel 11:** Körperpuder

| INCI Name | Produktname | Gew-% | Lieferant |
|---|---|---|---|
| *Phase A* | | *100.00* | |
| Mica | Silk Mica | 42.20 | www.vwr.com |
| Talc | Talc Powder | 18.00 | www.riedeldehaen.com |
| Boron Nitride | Softouch CCS 102 | 5.00 | www.advceramics.com |
| Nylon-12 | Orgasol 2002 D/Nat | 8.00 | www.atofinachemicals.c om |
| Magnesium Stearate | Magnesium Stearate | 6.00 | www.sigmaaldrich.com |
| Methylparaben, Propylparaben | Rokonsal SSH-1 | 0.30 | www.biochema.com |
| Mica (and) Iron Oxides | Prestige Soft Bronze | 9.00 | www.eckart.net |

(fortgesetzt)

| INCI Name | Produktname | Gew-% | Lieferant |
|---|---|---|---|
| *Phase A* | | *100.00* | |
| **Mehrschichtperl-glanzpigment** | **Mehrschichtperl-glanzpigment** | **0.50** | |
| Mica (and) Titanium Dioxide | Prestige Magic Orange | 9.00 | www.eckart.net |
| *Phase B* | | | |
| Tridecyl Stearate (and) Tridecyl Trimellitate (and) Dipentaerythrityl Hexacaprylate/Hexaca prate | Lipovol MOS-130 | 2.00 | www.lipochemicals.com |

Das Mehrschichtperlglanzpigment kann in einem Bereich von 0,2 - 5,0 Gew.-% eingesetzt werden. Der Ausgleich kann mit Mica erfolgen.

Die Inhaltsstoffe der Phase A wurden zusammengemischt, anschließend wurde Phase B zu Phase A zugegeben. Nach Vermischen in ein Gefäß abfüllen.

**Beispiel 12:** Lip Gloss

| INCI Name | Produktname | Gew-% | Lieferant |
|---|---|---|---|
| *Phase A* | | *100.00* | |
| Hydrogenated Polyisobutene (and) Ethylene/Propylene/Styrene Copolymer (and) Butylene/Ethylene/Styrene Copolymer | Versagel ME 750 | 78.90 | www.penreco. com |
| Simmondsia Chinensis (Jojoba) Seed Oil | Jojoba Oil - Natural/Golden | 2.00 | www.biochemica.c om |
| Caprylyl Trimethicone | Silcare Silicone 31 M50 | 7.00 | www.clariant.com |
| Stearyl Dimethicone | Silcare Silicone 41 M65 | 3.20 | www.clariant.com |
| Hydrogenated Polydecene | Nexbase 2002 | 4.00 | www.jandekker. com |
| Isopropyl Myristate | Isopropyl Myristate | 4.50 | www.vwr.com |
| *Phase B* | | | |
| **Mehrschichtperl-glanzpigment** | **Mehrschichtperl-glanzpigment** | **0.20** | |
| Propylparaben | Propyl-4-hydroxybenzoat | 0.20 | www. sigmaaldrich.com |

Das Mehrschichtperlglanzpigment kann in einem Bereich von 0,01 - 0,50 Gew.-% eingesetzt werden. Der Ausgleich kann mit Versagel ME 750 erfolgen.

Phase A wurde auf 85°C erhitzt, anschließend wurden die Inhaltstoffe der Phase B einzeln zu Phase A hinzugegeben und gerührt bis eine gleichmäßige Konsistenz entstand und dann in ein Lip Gloss Gefäß abgefüllt.

**Patentansprüche**

1. Mehrschichtperlglanzpigmente, umfassend mit optisch wirksamer Beschichtung versehene plättchenförmige transparente Substrate,
   **dadurch gekennzeichnet,**
   **dass** die optisch wirksame Beschichtung wenigstens

   (a) eine nicht absorbierende hochbrechende Schicht A mit einem Brechungsindex $n \geq 1{,}8$,
   (b) eine niedrigbrechende Schicht B mit einem Brechungsindex $n < 1{,}8$,

(c) eine nicht absorbierende hochbrechende Schicht C mit einem Brechungsindex n ≥ 1,8
sowie
(d) optional wenigstens eine äußere Schutzschicht D

umfasst und dass die Mehrschichtperlglanzpigmente eine Summenhäufgkeitsverteilung einer volumengemittelten Größenverteilungsfunktion mit den Kennzahlen $D_{10}$, $D_{50}$, $D_{90}$ und einem Span $\Delta D$ in einem Bereich von 0,7 -1,4 aufweisen, wobei der Span $\Delta D$ gemäß Formel (I)

$$\Delta D = (D_{90} - D_{10})/ D_{50} \qquad (I)$$

berechnet ist, mit der Maßgabe dass bei der Verwendung von Glasflakes als plättchenförmige transparente Substrate die mittlere geometrische Diese der Glasflakes in einem Bereich von 100 nm bis 700 nm liegt.

2. Mehrschichtperlglanzpigmente nach Anspruch 1,
   **dadurch gekennzeichnet,**
   **dass** die Mehrschichtperlglanzpigmente keine silberne Interferenzfarbe aufweisen.

3. Mehrschichtperlglanzpigmente nach einem der vorstehenden Ansprüche,
   **dadurch gekennzeichnet,**
   **dass** die Schicht B nicht absorbierend ist.

4. Mehrschichtperlglanzpigmente nach einem der vorstehenden Ansprüche,
   **dadurch gekennzeichnet,**
   **dass** die Mehrschichtperlglanzpigmente einen Span $\Delta D$ in einem Bereich von 0,7 bis 1,3 aufweisen.

5. Mehrschichtperlglanzpigmente nach einem der vorstehenden Ansprüche,
   **dadurch gekennzeichnet,**
   **dass** die optische Schichtdicke der Schicht A in einem Bereich von 30 nm bis 900 nm liegt.

6. Mehrschichtperlglanzpigmente nach einem der vorstehenden Ansprüche,
   **dadurch gekennzeichnet,**
   **dass** die optische Schichtdicke der Schicht B in einem Bereich von 30 nm bis 500 nm liegt.

7. Mehrschichtperlglanzpigmente nach einem der vorstehenden Ansprüche,
   **dadurch gekennzeichnet,**
   **dass** die optische Schichtdicke der Schicht B in einem Bereich von 30 nm bis ≤ 150 nm liegt.

8. Mehrschichtperlglanzpigmente nach einem der vorstehenden Ansprüche,
   **dadurch gekennzeichnet,**
   **dass** die optische Schichtdicke der Schicht C in einem Bereich von 30 nm bis 900 nm liegt.

9. Mehrschichtperlglanzpigmente nach einem der vorstehenden Ansprüche,
   **dadurch gekennzeichnet,**
   **dass** die Schichten A und C jeweils Titanoxid, vorzugsweise Titandioxid, umfassen.

10. Mehrschichtperlglanzpigmente nach einem der vorstehenden Ansprüche,
    **dadurch gekennzeichnet,**
    **dass** die Schicht B Siliziumoxid, vorzugsweise Siliziumdioxid, umfasst.

11. Mehrschichtperlglanzpigmente nach einem der vorstehenden Ansprüche,
    **dadurch gekennzeichnet,**
    **dass** die plättchenförmigen Substrate aus der Gruppe, bestehend aus natürlichem Glimmer, synthetischem Glimmer, Glasflakes, $SiO_2$-Plättchen, $Al_2O_3$-Plättchen und deren Gemische, ausgewählt ist

12. Verfahren zum Herstellen der Mehrschichtperlglanzpigmente nach einem der vorstehenden Ansprüche,
    **dadurch gekennzeichnet,**

**dass** das Verfahren folgende Schritte umfasst:

(i) Größenklassieren der zu beschichtenden plättchenförmigen transparenten Substrate, so dass die zu beschichtenden plättchenförmigen transparenten Substrate eine volumengemittelte Größenverteilungsfunktion mit den Kennzahlen $D_{10}$, $D_{50}$, $D_{90}$ und einem Span $\Delta D$ in einem Bereich von 0,7 -1,4 aufweisen, wobei der Span $\Delta D$ gemäß der Formel $\Delta D = (D_{90} - D_{10})/ D_{50}$ definiert ist,
(ii) Aufbringen wenigstens der Schichten A bis C auf die plättchenförmigen transparenten Substrate sowie optional wenigstens einer Schicht D,
oder

(iii) Aufbringen wenigstens der Schichten A bis C auf die plättchenförmigen transparenten Substrate sowie optional wenigstens einer Schicht D,
(iv) Größenklassieren der zu beschichtenden plättchenförmigen transparenten Substrate, so dass die zu beschichtenden plättchenförmigen transparenten Substrate eine volumengemittelte Größenverteilungsfunktion mit den Kennzahlen $D_{10}$, $D_{50}$, $D_{90}$ und einem Span $\Delta D$ in einem Bereich von 0,7-1,4

aufweisen, wobei der Span $\Delta D$ gemäß der Formel $\Delta D = (D_{90} - D_{10})/D_{50}$ definiert ist.

13. Verwendung der Mehrschichtperlglanzpigmente nach einem der Ansprüche 1 bis 11 in kosmetischen Formulierungen, Kunststoffen, Folien, Textilien, keramischen Materialien, Gläsern und Beschichtungszusammensetzungen wie Farben, Druckfarben, Lacken oder Pulverlacken.

14. Gegenstand,
**dadurch gekennzeichnet,**
**dass** der Gegenstand die Mehrschichtperlglanzpigmente nach einem der Ansprüche 1 bis 11 aufweist oder enthält.

15. Zubereitung,
**dadurch gekennzeichnet,**
**dass** die Zubereitung die Mehrschichtperlglanzpigmente nach einem der Ansprüche 1 bis 11 aufweist oder enthält.

**Claims**

1. Multi-layered pearlescent pigments, comprising platelet-shaped transparent substrates provided with an optically active coating, **characterised in that**
the optically active coating comprises at least

(a) a non-absorbing high-index layer A with a refractive index n $\geq$1.8,
(b) a low-index layer B with a refractive index n < 1.8,
(c) a non-absorbing high-index layer C with a refractive index n $\geq$1.8,
and
(d) optionally, at least one outer protective layer D,

and the multi-layered pearlescent pigments have a cumulative frequency distribution of a volume-averaged size distribution function with the indices $D_{10}$, $D_{50}$, $D_{90}$ and a span $\Delta D$ in a range of 0.7 - 1.4, the span $\Delta D$ being calculated on the basis of formula (I)

$$\Delta D = (D_{90} - D_{10})/D_{50} \qquad (I)$$

provided that, if using glass flakes as platelet-shaped transparent substrates, the mean geometric thickness of the glass flakes is within a range of 100 nm to 700 nm.

2. Multi-layered pearlescent pigments as claimed in claim 1,
**characterised in that**
the multi-layered pearlescent pigments do not have a silver interference colour.

3. Multi-layered pearlescent pigments as claimed in one of the preceding claims,

**characterised in that**
layer B is non-absorbing.

4. Multi-layered pearlescent pigments as claimed in one of the preceding claims,
   **characterised in that**
   the multi-layered pearlescent pigments have a span $\Delta D$ within a range of 0.7 to 1.3.

5. Multi-layered pearlescent pigments as claimed in one of the preceding claims,
   **characterised in that**
   the optical layer thickness of layer A is within a range of 30 nm to 900 nm.

6. Multi-layered pearlescent pigments as claimed in one of the preceding claims,
   **characterised in that**
   the optical layer thickness of layer B is within a range of 30 nm to 500 nm.

7. Multi-layered pearlescent pigments as claimed in one of the preceding claims,
   **characterised in that**
   the optical layer thickness of layer B is within a range of 30 nm to# 150 nm.

8. Multi-layered pearlescent pigments as claimed in one of the preceding claims,
   **characterised in that**
   the optical layer thickness of layer C is within a range of 30 nm to 900 nm.

9. Multi-layered pearlescent pigments as claimed in one of the preceding claims,
   **characterised in that**
   layers A and C respectively comprise titanium oxide, preferably titanium dioxide.

10. Multi-layered pearlescent pigments as claimed in one of the preceding claims,
    **characterised in that**
    layer B comprises silicon oxide, preferably silicon dioxide.

11. Multi-layered pearlescent pigments as claimed in one of the preceding claims,
    **characterised in that**
    the platelet-shaped substrate is selected from the group comprising natural mica, synthetic mica, glass flakes, $SiO_2$ platelets, $Al_2O_3$ platelets and mixtures thereof.

12. Method of producing multi-layered pearlescent pigments as claimed in one of the preceding claims,
    **characterised in that**
    the method comprises the following steps:

    (i) size classifying the platelet-shaped transparent substrates to be coated so that the platelet-shaped transparent substrates to be coated have a volume-averaged size distribution function with the characteristics $D_{10}$, $D_{50}$, $D_{90}$ and a span $\Delta D$ in a range of 0.7 - 1.4, the span $\Delta D$ being defined on the basis of the formula $\Delta D = (D_{90} - D_{10})/D_{50}$,
    (ii) applying at least layers A to C to the platelet-shaped transparent substrates and optionally at least one layer D, or
    (iii) applying at least layers A to C to the platelet-shaped transparent substrates and optionally at least one layer D,
    (iv) size classifying the platelet-shaped transparent substrates to be coated so that the platelet-shaped transparent substrates to be coated have a volume-averaged size distribution function with the characteristics $D_{10}$, $D_{50}$, $D_{90}$ and a span $\Delta D$ in a range of 0.7 - 1.4, the $\Delta D$ span being defined on the basis of the formula $\Delta D = (D_{90} - D_{10})/D_{50}$.

13. Use of the multi-layered pearlescent pigments as claimed in one of claims 1 to 11 in cosmetic formulations, plastics, foils, textiles, ceramic materials, glasses and coating compositions such as paints, printing inks, lacquers or powder coatings.

14. Object,
    **characterised in that**
    the object incorporates or contains the multi-layered pearlescent pigments as claimed in one of claims 1 to 11.

**15.** Preparation,
**characterised in that**
the preparation incorporates or contains the multi-layered pearlescent pigments as claimed in one of claims 1 to 11.

**Revendications**

**1.** Pigments perlés multicouches, comprenant des substrats transparents en forme de plaquettes dotés d'un revêtement optiquement actif,
**caractérisés en ce que**
le revêtement optiquement actif comprend au moins

(a) une couche A non absorbante à haut indice de réfraction avec un indice de réfraction $n \geq 1,8$
(b) une couche B à bas indice de réfraction avec un indice de réfraction $n < 1,8$,
(c) une couche C non absorbante à haut indice de réfraction avec un indice de réfraction $n \geq 1,8$,
de même que
(d) en option, au moins un couche de protection extérieure D,

et **en ce que** les pigments perlés multicouches présentent une répartition de somme de fréquence d'une fonction de répartition de grandeur par moyenne volumique avec les valeurs caractéristiques $D_{10}$, $D_{50}$, $D_{90}$ et un $\Delta D$ de copeau dans un intervalle de 0,7 à 1,4, le $\Delta D$ de copeau étant calculé conformément à la formule

$$\Delta D = (D_{90} - D_{10})/D_{50} \qquad (I)$$

avec la condition que lors de l'utilisation de plaquettes de verre comme substrats transparents en forme de plaquettes, l'épaisseur géométrique moyenne des plaquettes de verre est comprise dans un intervalle de 100 nm à 700 nm.

**2.** Pigments perlés multicouches selon la revendication 1,
**caractérisés en ce que**
les pigments perlés multicouches ne présentent pas de couleur d'interférence argentée.

**3.** Pigments perlés multicouches selon l'une quelconque des revendications précédentes,
**caractérisés en ce que**
la couche B n'est pas absorbante.

**4.** Pigments perlés multicouches selon l'une quelconque des revendications précédentes,
**caractérisés en ce que**
les pigments perlés multicouches présentent un $\Delta D$ de copeau dans un intervalle de 0,7 à 1,3.

**5.** Pigments perlés multicouches selon l'une quelconque des revendications précédentes,
**caractérisés en ce que**
l'épaisseur de couche optique de la couche A est comprise dans un intervalle de 30 nm à 900 nm.

**6.** Pigments perlés multicouches selon l'une quelconque des revendications précédentes,
**caractérisés en ce que**
l'épaisseur de couche optique de la couche B est comprise dans un intervalle de 30 nm à 500 nm.

**7.** Pigments perlés multicouches selon l'une quelconque des revendications précédentes,
**caractérisés en ce que**
l'épaisseur de couche optique de la couche B est comprise dans un intervalle de 30 nm à $\leq$ 150 nm.

**8.** Pigments perlés multicouches selon l'une quelconque des revendications précédentes,
**caractérisés en ce que**
l'épaisseur de couche optique de la couche C est comprise dans un intervalle de 30 nm à 900 nm.

**9.** Pigments perlés multicouches selon l'une quelconque des revendications précédentes,

**caractérisés en ce que**
les couches A et C comprennent respectivement de l'oxyde de titane, de préférence du dioxyde de titane.

10. Pigments perlés multicouches selon l'une quelconque des revendications précédentes,
**caractérisés en ce que**
la couche B comprend de l'oxyde de silicium, de préférence du dioxyde de silicium.

11. Pigments perlés multicouches selon l'une quelconque des revendications précédentes,
**caractérisés en ce que**
les substrats en forme de plaquettes sont choisis parmi le groupe constitué du mica naturel, du mica synthétique, des plaquettes de verre, des plaquettes de $SiO_2$, des plaquettes de $Al_2O_3$ et de leurs mélanges.

12. Procédé de préparation des pigments perlés multicouches selon l'une quelconque des revendications précédentes,
**caractérisé en ce que**
le procédé comprend les étapes suivantes :

(i) triage par tailles des substrats transparents en forme de plaquettes devant être revêtus, de telle sorte que les substrats transparents en forme de plaquettes devant être revêtus présentent une fonction de répartition de grandeur par moyenne volumique avec les valeurs caractéristiques $D_{10}$, $D_{50}$, $D_{90}$ et un $\Delta D$ de copeau dans un intervalle de 0,7 à 1,4, le $\Delta D$ de copeau étant défini conformément à la formule $\Delta D = (D_{90} - D_{10})/D_{50}$,
(ii) application d'au moins les couches A à C sur les substrats transparents en forme de plaquettes ainsi qu'en option au moins une couche D,
ou
(iii) application d'au moins les couches A à C sur les substrats transparents en forme de plaquettes ainsi qu'en option au moins une couche D,
(iv) triage par tailles des substrats transparents en forme de plaquettes devant être revêtus, de telle sorte que les substrats transparents en forme de plaquettes devant être revêtus présentent une fonction de répartition de grandeur par moyenne volumique avec les valeurs caractéristiques $D_{10}$, $D_{50}$, $D_{90}$ et un $\Delta D$ de copeau dans un intervalle de 0,7 à 1,4, le $\Delta D$ de copeau étant défini conformément à la formule $\Delta D = (D_{90} - D_{10})/D_{50}$.

13. Utilisation de pigments perlés multicouches selon l'une quelconque des revendications 1 à 11 dans des formulations cosmétiques, des plastiques, des films/feuilles, des textiles, des matériaux céramiques, des verres et des compositions de revêtement telles des peintures, des encres d'imprimerie, des laques ou des poudres pour revêtements électrostatiques.

14. Objet
**caractérisé en ce que**
l'objet présente ou contient les pigments perlés multicouches selon l'une quelconque des revendications 1 à 11.

15. Préparation
**caractérisée en ce que**
la préparation présente ou contient les pigments perlés multicouches selon l'une quelconque des revendications 1 à 11.

Abbildung 1:

Abbildung 2: Beeinflussung der Laserbeugung durch Partikeleigenschaften

d = Partikeldurchmesser

Partikeleigenschaften:

d: Durchmesser

1:Beugung

2: Brechung

3 Reflexion

4: Absorption

Abbildung 3: Farbverlauf der äußeren TiO$_2$-Beschichtung

**Farbverlauf während der äußeren TiO$_2$-Beschichtung**
**Multilayer Aufbau: TiO$_2$/SiO$_2$/TiO$_2$**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0753545 B2
- WO 2004067645 A2
- WO 2006088759 A1
- EP 0948572 B1
- JP 07246366 B
- EP 1025168 B1
- WO 2003006558 A2
- WO 2004055119 A1
- WO 2002090448 A2
- WO 2006110359 A2
- US 2006042509 A1
- WO 2009103322 A1
- EP 0289240 A1
- WO 2004056716 A1
- WO 2005063637 A1
- EP 1980594 B1
- WO 2006021386 A1

**In der Beschreibung aufgeführte Nicht-Patentliteratur**

- *Byk-Gardner Katalog,* 2007, 14
- *Gerät: MALVERN Mastersizer,* 2000